# EUROPEAN PATENT SPECIFICATION

(11) **EP 4 140 512 B1**
(45) Date of publication and mention of the grant of the patent: **19.08.2026**
(21) Application number: 22168980.5
(22) Date of filing: 20.04.2022
(51) Int. Cl.: A61L 27/54, A61L 31/16, A61L 27/06, A61L 27/34, A61L 31/02, A61L 31/10

(54) **NEW COATING OF IMPLANTS**
NEUE BESCHICHTUNG VON IMPLANTATEN
NOUVEAU REVÊTEMENT D'IMPLANTS

(30) Priority: 27.08.2021 EP 21193637
(43) Date of publication of application: 01.03.2023
(73) Proprietor: Universität Münster, 48149 Münster (DE)
(72) Inventor: Schulze, Martin, 48149 Münster (DE); Fobker, Manfred, 48149 Münster (DE); Schulte, Erhard, 48149 Münster (DE); Gosheger, Georg, 48149 Münster (DE); Pützler, Jan, 48149 Münster (DE)
(74) Representative: Schiweck Weinzierl Koch Patentanwälte Partnerschaft mbB

(56) References cited:
- EP-B1- 0 328 421
- WO-A1-2023/025944
- US-A1- 2016 166 738
- US-B2- 6 949 598

## Description

### TECHNICAL FIELD OF THE INVENTION

The present invention relates to an implant having a surface comprising a coating on at least a portion of the surface of the implant, wherein the coating comprises a first layer, the first layer comprising a polylactide and silver ions. The present invention further relates to a method of manufacturing the implant as well as to an implant obtainable by that method.

### BACKGROUND ART

Implants and endoprostheses are always exposed to a risk of colonization by pathogens as germs, bacteria and biofilm formation after insertion into the human body, so that infections, usually mixed infections, occur in 0.5 to 2% of cases (Ong et al. 2009; Gbejuade et al. 2015). With the implant volume and depending on the implantation location, the risk of infection increases. This explains why the infection rate in tumor prostheses or megaprostheses for the reconstruction of a usually pronounced defect route is significantly higher risk of infection of up to 20% (Gosheger et al. 2006; Funovics et al. 2011). If there are further complications, the risk increases to about 40% with secondary revision procedures (Theil et al. 2019).

In tumor orthopedics, soft tissue reconstruction often includes knitted tubes, so-called connecting tubes, e.g., the Trevira (Mutars^{®}, Implantcast Corp, Buxtehude, Germany) from polyethylene terephthalate. Due to their net-like structure, these result in a potentiation of the surface and thus of the attack surface. Infections can lead to amputations or sepsis with a lethal outcome in acute courses. Tumor patients are particularly affected by this risk, as they often have limited immune competence, e.g., due to necessary chemotherapy or radiotherapy.

The therapy of the prosthetic infection includes both surgical rehabilitation and anti-infective therapy. This usually takes place systemically over several weeks and is supplemented topically, e.g., by introducing antibiotic-bearing powders or placeholders made of bone cement and spherical chains as active ingredient carriers. Bone cement used is usually a plastic polymethylmethacrylate (PMMA) mixed from powder and liquid activator, which becomes a malleable mass after mixing and cures completely within a few minutes. In everyday life, this material is known as plexiglass.

Antibiotic powders can cause tissue damage due to their high contact surface and the resulting locally high concentrations. This also applies to other substances such as virustatics and fungicides. The selection pressure on the pathogens is increased and resistance can potentially develop. Due to their side-effect profile, locally applied pharmaceuticals also carry the risk of temporary or even permanent organ damage. Antibiotic powders are often applied as additive to PMMA. The release from these PMMA carriers is very limited. The active ingredient is embedded into the carrier material, so to speak. Since this material is not absorbed in the human body, the concentration of the active ingredient drops quickly after introduction. Further release is only possible due to material damage, such as abrasion, on the carrier material. Other anti-infective substances include metals such as silver. These have a bactericidal and bacteriostatic effect. However, when introduced into the body as a powder or in high concentrations, it has potentially tissue- and cell-toxic effects. In order to take advantage of the positive properties, silver coatings on implants have been developed in the past (Gosheger and Sass 2002).

The development of anti-infective coatings, e.g., with antibiotics and compounds with other materials, have so far only been able to demonstrate short-term efficacy (Romanò et al. 2015). Even anti-infective metallic coatings such as e.g., with silver have already achieved a significant reduction in the infection rate of between 7 and 12 % (Hardes et al. 2010; Schmidt-Braekling et al. 2017). However, control over the concentration and duration of release within the scope of elution kinetics is not yet possible (Scoccianti et al. 2016).

EP 0328 421 B1 discloses infection-resistant compositions, medical devices and surfaces and methods for preparing and using same.

Thus, there is a need for further coatings for implants.

### SUMMARY OF THE INVENTION

In a first aspect, the present invention relates to an implant having a surface comprising a coating on at least a portion of the surface of the implant, wherein the coating comprises
a) a first layer, the first layer comprising at least one polylactide and silver ions and
   wherein
   i) the silver ions are homogenously dissolved in the first layer and
   ii) optionally a second layer is present arranged on the first layer, wherein the second layer comprises at least one lipid and at least one antibiotic/antiinfective,

   wherein the at least one lipid is a monoacylglycerol, a diacyglycerol, a triacylglycerol, sterol, saturated and/or unsaturated fatty acid, saturated and/or unsaturated fatty acid-salts, and/or saturated fatty alcohol, and/or saturated fatty acid ester and/or a combination thereof;
   the monoacylglycerol comprises glycerol and wherein one of the three available hydroxy groups are esterified with a saturated fatty acid;
   the diacyglycerol comprises glycerol wherein two of the three available hydroxy groups are esterified with a saturated fatty acid;
   the triacylglycerol comprises glycerol wherein three of the three available hydroxy groups are esterified with a saturated fatty acid;
   the fatty acid is R-COOH or the corresponding fatty acid salt thereof;
   the fatty alcohol R-OH;
   the fatty acid ester R-OR;
   wherein R is (C₆-₂₃)alkyl or (C₆-₂₃)alkenyl.

In a second aspect, the present invention relates to a method of manufacturing an implant, preferably according to the first aspect, wherein the method comprises forming a first layer arranged on at least a portion of a surface of the implant, said forming of the first layer comprising contacting at a least a portion of the surface of the implant with a first coating composition comprising a polylactide and silver ions and wherein the coating composition comprises pyridine.

In a third aspect, the present invention relates to an implant, obtainable by a method as described above.

The present invention provides a coating for implants which allows the release of silver ions as antibiotic effect from the implant after implantation, in respect to the released concentration over time in a controlled manner. In one embodiment, the implanted implant may be subjected to a shock wave which triggers release of a high concentration of silver ions in a short period of time, in order to prevent or to treat a local infection near the implant. The shock wave application may be repeated.

In one embodiment, on the first coating, comprising the silver ions, a second coating may be present. In contrast to other developments, for example nanosilver particles, due to their precious metal properties, are released only in very small amounts from such layers when enclosed by a polymer - or quickly surround themselves with a poorly soluble silver chloride/protein layer. The second coating may comprise a further antibiotic compound. Preferably, the further antibiotic compound is released in a linear fashion with a constant concentration. Thus, initial high and toxic concentrations are avoided. Furthermore, the concentration of the antibiotic compound does not fall below the minimal inhibitory concentration up to months, and the development of pathogens resistant to the antibiotic compound may be avoided. Further application of multiple layers to combine the release kinetics is possible.

### BRIEF DESCRIPTION OF THE FIGURES

Figure 1 EDS-linescan for the Element Ag on a cross-section polish of the PLLA+Ag coating. A homogeneous distribution of the silver both longitudinally and transversely to the coating is evident.
Figure 2 Examplary embodiment of the implant, method, and coating for use of the invention.
Figure 3 EDX analysis of the oxalic acid etching and subsequent washing with calcium lactate solution. (a) Scanning electron micrograph of the surface at 38x magnification with HV = 20 kV and WD = 17.7mm. (b) EDX analysis of the element calcium to verify complete and homogeneous wetting of the surface.
Figure 4 Results from contact angle measurement (top) and resulting surface free energy (bottom).
Figure 5 Presentation of the experimental setup for the evaluation of the activatability of the RESOMER^{®} L coating via focused extracorporeal shock wave. The sterilized LDPE sample bag is not displayed to increase clarity. (a) Experimental setup with adapted laser pointer for position determination. The laser beam (arrow) shows the focus area of the shock wave on the test specimen. (b) Experimental setup with adapted shock wave device. The ellipsoid (*) represents the focus area (dashed arrow) of the shock wave.
Figure 6 Sections of the experimental setup from Figure 5(b) with designation of the relevant components and dimensions (sample bag is not shown).
Figure 7 REM-/EDX-analysis of the coating after the application of extracorporeal shock waves. (a) Coating in the initial state. (b) Sample surface (Ti₆Al₄V) in the range of applied shock waves. (c) Disruption of the coating by the shock waves. (d) Heat map of titanium concentration at the surface. The silver contents given in (a) and (b) show the almost complete release of the silver contained in the coating.
Figure 8 Flow chamber test: Schematic experimental setup; Sample P is rinsed at 37°C with buffer or blood at a flow rate of 1 ml/h, and the fractions are collected over 6 weeks and the active ingredient in them is measured.
Figure 9 Release kinetics of rifampin from a coating with TAG / PLGA (•) and lipid coating alternatives as cholesterol (▲) and diacylglycerol (+). The curves for cholesterol and diacylglycerol proof that the release kinetics can be modified by altering the manufacturing process of the coating. The dashed red line indicates the MIC for S. *aureus* with 0.06 µg/mL.
Figure 10 Rifampin-Eluate with different concentrations of maltotriose in the inhibition test.
Figure 11 Osteoblast growth in contact with titanium implant sample without and with TAG-coating after 1 / 3 / 5 days.
Figure 12 Growth curves *S. aureus* in contact with supernatants of coatings with Ag 8% (1A), 4% (2A), 2% (3A) after shock wave application compared to control (no substance).
Figure 13 Growth curves *E. coli* in contact with supernatants of coatings with Ag 8% (1A), 4% (2A), 2% (3A) after shock wave application compared to control (no substance).
Figure 14 Colony forming units (cfu) of *S*. *aureus 6850* in contact with supernatants after shock wave application to coatings containing Ag 8% (1A), 4% (2A), 2% (3A) compared to control.
Figure 15 Colony forming units (cfu) of *E. coli TG1* in contact with supernatants after shock wave application to coatings containing Ag 8% (1A), 4% (2A), 2% (3A) compared to control.
Figure 16 WST-1 assay with incubation of fibroblasts in contact with the titanium implant sample, PLLA coating with a silver content of 0 %, 2 %, 4 % and 8 % in comparison to the control fibroblasts (incubation time in minutes) after ESW application.
Figure 17 The silver released by the shock wave test was tested for its biological activity in a suspension culture (Figure 11A) and in an inhibitory test (Figure 11B). Depending on the concentration, the three different silver layers (1,2,3) showed a bactericidal effect in both S. *epidermidis RP62A* (upper row A) and *S. aureus 6850* (lower row B), which could also be quantified turbidimetrically. Also, in the inhibitory test with *S. aureus 6850,* the silver layers used show microbiological activity in contrast to the control K (pure polylactide).
Figure 18 Left: bacterial count for *S*. *aureus* was only 2.78x10E6 compared to 7.71x10E7 of the control (without supernatant); Right: for *E. coli* a complete eradication compared to 2.94x10E7 of the control.
Figure 19 Bacterial count including standard deviation for *S*. *epidermidis* when incubated with TAG-coatings containing different amounts of vancomycin for 24 h. 2 % vancomycin already leads to a significant reduction of CFU compared to the control, Ti6Al4V and TAG coating. For the TAG coating containing 10 % vancomycin, a complete eradication of S. *epidermidis* is visible. (*: p < 0.05, **: p < 0.01).
Figure 20 Light microscopic (LM) bright field images of the TAG coating surface at magnifications of (a) 100x, (b) 200x and (c) 500x. Red circle indicates two pores.
Figure 21 SEM image of the PLLA coating surface. The images (a)-(c) show a homogeneously coated area at (a) 500x, (b) 2000x and (c) 5000x magnification. The white arrow indicates a residual from preparation, which is not part of the coating.
Figure 22 Left: confocal microscopically determined surface roughness of the components of the coating system. (*: p < 0.05, **: p < 0.01), Right: Confocal microscope images for determining the surface roughness.
Figure 23 LM bright field cross-section images at 500x magnification of (a) PLLA coating, (b) TAG coating and (c) bilayer coating (PLLA+TAG).
Figure 24 SEM image of the cross-section of the PLLA coating released from the titanium surface.
Figure 25 Coating thickness of the first layer coating (PLLA), second layer coating (TAG) and bilayer coating (PLLA+TAG) measured microscopically on cross-section polish.

### DETAILED DESCRIPTION OF THE INVENTION

The solution of the present invention is described in the following, exemplified in the appended examples, illustrated in the Figures and reflected in the claims.

It is noted that as used herein, the singular forms "a", "an", and "the", include plural references unless the context clearly indicates otherwise. Thus, for example, reference to "a reagent" includes one or more of such different reagents and reference to "the method" includes reference to equivalent steps and methods known to those of ordinary skill in the art that could be modified or substituted for the methods described herein.

Unless otherwise indicated, the term "at least" preceding a series of elements is to be understood to refer to every element in the series. Those skilled in the art will recognize, or be able to ascertain using no more than routine experimentation, many equivalents to the specific embodiments of the invention described herein. Such equivalents are intended to be encompassed by the present invention.

The term "and/or" wherever used herein includes the meaning of "and", "or" and "all or any other combination of the elements connected by said term".

The term "less than" or in turn "more than" does not include the concrete number. For example, less than 20 means less than the number indicated. Similarly, more than or greater than means more than or greater than the indicated number, f.e. more than 80 % means more than or greater than the indicated number of 80 %.

Throughout this specification and the claims which follow, unless the context requires otherwise, the word "comprise", and variations such as "comprises" and "comprising", will be understood to imply the inclusion of a stated integer or step or group of integers or steps but not the exclusion of any other integer or step or group of integer or step. When used herein the term "comprising" can be substituted with the term "containing" or "including" or sometimes when used herein with the term "having". When used herein "consisting of" excludes any element, step, or ingredient not specified.

The term "including" means "including but not limited to". "Including" and "including but not limited to" are used interchangeably.

It should be understood that this invention is not limited to the particular methodology, protocols, material, reagents, and substances, etc., described herein and as such can vary.

The invention is directed to an implant having a surface comprising a coating on at least a portion of the surface of the implant.

An intermediate may be arranged between the surface of the implant and the first layer. Preferably, the intermediate layer is a coating or pretreatment. More preferably, the intermediate layer is selected from the group consisting of PVD-layers (physical vapor deposition), TPS-layers (titanium plasma spray), calcium phosphate layers, layers caused by etching (oxidation, passivation, roughening), anodization, and a silver metal layer or any combination thereof.

The implant may be any implant suitable for implantation in the human or animal body. Preferably, the implant is a medical implant. More preferably, the implant is selected from a group comprising an implantable prosthesis, in particular a hip prosthesis, a shoulder prosthesis, an elbow prosthesis, a knee prosthesis, or an implant for trauma, maxillo-facial, or spinal surgery such as e.g. a screw or a plate, a nail or a rod, intervertebral spacers, as well as others including but not limited to cardiological and neurosurgical implants (shunts, electrodes, valves, stents, catheters, etc.), most preferably, the implant is selected from a group comprising an implantable prosthesis, in particular a hip prosthesis, a shoulder prosthesis, an elbow prosthesis, a knee prosthesis, or an implant for trauma surgery such as e.g. a screw or a plate.

The implant and/or the surface of the implant may comprise any material suitable for implants. The implant and/or the surface may comprise a metal, and/or a non-metal such as a polymer. Preferably, the implant and/or the surface comprise a metal. Preferably, the implant and/or the surface material comprise a metal such as 316 LVM implant steel, TiN coated Cr-Co-Mo, CoCrMo, titanium and/or a titanium alloy. More preferably, the titanium alloy is Ti₆Al₄v.

In one embodiment, at least the surface of the implant is made of metal. Preferably, the surface material comprises a metal such as 316 LVM implant steel, TiN coated Cr-Co-Mo, CoCrMo, titanium or a titanium alloy. More preferably, the titanium alloy is Ti₆Al₄V.

Preferably, the implant and/or the surface of the implant comprises at least 30 wt.-%, more preferably at least 60 wt.-%, most preferably at least 80 wt.-%, particular preferred at least 90 wt.-%, most particular preferred at least 95 wt.-% of a metal based on the overall weight of the implant and/or surface of the implant.

Preferably, the implant and/or the surface of the implant comprises less than 30 wt.-%, more preferably less than 60 wt.-%, most preferably less than 80 wt.-%, particular preferred less than 90 wt.-%, most particular preferred less than 95 wt.-% of a metal based on the overall weight of the implant and/or surface of the implant.

In one embodiment, at least the portion of surface of the implant which is coated is made of metal. Preferably, the surface material comprises titanium or a titanium alloy. Preferably, the titanium alloy is Ti₆Al₄V.

Preferably, at least 10%, more preferably at least 40%, most preferably at least 60%, particular preferred at least 90% and much more preferred 100% of the surface of the implant is coated.

In one embodiment, at least the surface of the implant is made of polymer. Preferably, the implant and/or the surface material comprises a polymer consisting of the group polyether ether ketone (PEEK), ultra-High molecular weight polyethylene (UHMWPE), low density polyethylene (LDPE), polymethylmethacrylate (PMMA), or polyphenylsulfone (PPSU). More preferably, the polymer is PEEK.

Preferably, the implant and/or the surface of the implant comprises at least 30 wt.-%, more preferably at least 60 wt.-%, most preferably at least 80 wt.-%, particular preferred at least 90 wt.-%, most particular preferred at least 95 wt.-% of a polymer based on the overall weight of the implant and/or surface of the implant.

Preferably, the implant and/or the surface of the implant comprises less than 30 wt.-%, more preferably less than 60 wt.-%, most preferably less than 80 wt.-%, particular preferred less than 90 wt.-%, most particular preferred less than 95 wt.-% of a polymer based on the overall weight of the implant and/or surface of the implant.

In one embodiment, at least the portion of surface of the implant which is coated is made of a polymer. Preferably, the surface material comprises PEEK. More preferably, the polymer is PEEK.

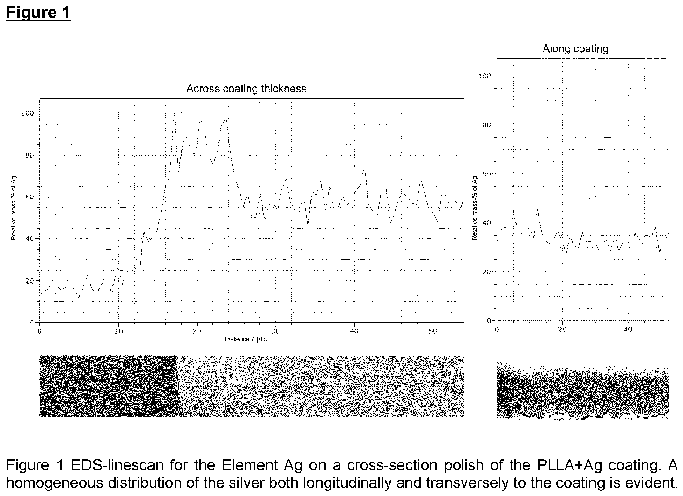

The coating comprises a first layer, the first layer comprising at least one polylactide and silver ions. The silver ions are homogeneously dissolved (figure 1).

Preferably, the first layer comprises at least one polylactide selected from the group consisting of poly(L-lactide) (CAS number: 33135-50-1), poly(D-lactide), poly(L-lactide-co-D,L-lactide) (CAS number: 52305-30-3), poly(D-lactide-co-glycolide), poly(L-lactide-co-glycolide) (CAS number: 30846-39-0), poly(L-lactide-co-caprolactone) (CAS number: 65408-67-5), and Poly(L-lactide-co-trimethylene carbonate) (CAS number: 113883-70-8), poly(D,L-lactide-co-glycolide) (CAS number: 1354955-03-5), more preferably poly(L-lactide).

Preferably, the first layer comprises at least 70%, more preferably at least 80%, most preferably at least 90%, particularly preferred at least 95%, more particularly preferred at least 99% of the at least one polylactide.

Preferably, the first layer comprises of from 0.01 to 20%, more preferably 0.1 to 15%, most preferably 0.5 to 10%, and particularly preferred 1.5 to 10%, particularly preferred 5 to 10 %, by weight silver ions, based on 100% by weight of the first layer.

The counterion of the silver compounds may be selected from a group of silver compounds that are soluble and non-toxic in non-polar solvents. More preferably, the counterion is selected from the group consisting of diethyldithiocarbamate, nitrate, benzoate, and any combination thereof.

The silver ions may be provided in form of a salt or a silver complex compound. Preferably, the silver ions are provided in form of a salt. More preferably, the silver salt is selected from a group consisting of silver sulfadiazine, silver diethyldithiocarbamate, silver oxide, silver carbonate and silver nitrate, silver acetate, silver benzoate, silver iodate, silver laurate, silver protein, silver chloride, and silver palmitate and any combination thereof, most preferably silver diethyldithiocarbamate, particularly preferred silver nitrate.

The first layer may further comprise hydroxyapatite or another poorly soluble calcium compound e.g. calcium carbonate. The first layer may comprise 0.1 to 10% by weight hydroxyapatite, based on 100% by weight of the first layer.

The first layer may further comprise phages. Preferably, the first layer comprises phages-enzymes, more preferably the phage-enzymes are endolysins, most preferably the endolysins are Staphefekt^{™} (Micreos, 3721 MA Bilthoven, Netherlands).

Preferably, the first layer comprises of from 0.01 to 20%, more preferably 0.1 to 15%, most preferably 0.5 to 10%, and particularly preferred 1.5 to 10%, particularly preferred 5 to 10 %, by weight phage-enzymes, based on 100% by weight of the first layer.

Preferably, the first layer has a thickness of from 1 to 50 µm.

In a further embodiment, the coating further comprises a second layer arranged on the first layer, wherein the second layer comprises an antibiotic/antiinfective. The second layer comprises a poly(lactide-co-glycolide) and/or at least one lipid.

In a further embodiment,
Preferably, the poly(lactide-co-glycolide) in the second layer is poly(D-lactide-co-glycolide), or poly(L-lactide-co-glycolide) (CAS number: 30846-39-0), or poly(D,L-lactide-co-glycolide) (CAS number: 1354955-03-5).

Preferably, the at least one lipid is a monoacylglycerol, a diacyglycerol, a triacylglycerol, sterol, saturated and/or unsaturated fatty acid, saturated and/or unsaturated fatty acid-salts, and/or saturated fatty alcohol, and/or saturated fatty acid ester and/or a combination thereof. Wherein the monoacylglycerol comprises glycerol wherein one of the three available hydroxy groups are esterified with a saturated fatty acid. Wherein the diacyglycerol comprises glycerol wherein two of the three available hydroxy groups are esterified with a saturated fatty acid. Wherein the triacylglycerol comprises glycerol wherein three of the three available hydroxy groups are esterified with a saturated fatty acid.

The fatty acid is preferably R-COOH or the fatty acid salt the corresponding salt thereof, or a fatty alcohol R-OH, or a fatty acid ester R-OR wherein R is (C₆-₂₃)alkyl or (C₆-₂₃)alkenyl. More preferably, the fatty acid is selected from palmitic acid, CAS number: 57-10-3), the fatty alcohol is selected from 1-hexadecanol, CAS number: 36653-82-4 and the fatty acid ester is selected from cetylpalmitate, CAS number: 540-10-3.

Sterol is an organic compound with formula C₁₇H₂₈O; whose structure is derived from that of gonane by replacement of a hydrogen atom in position 3 by a hydroxyl group. Preferably, the sterol is cholesterol, CAS number: 26657-95-4.

More preferably, the monoglycerol is selected from glycerol α-monolaurate, CAS number: 142-18-7.

More preferably, the diacyglycerol is selected from dipalmitin, CAS number: 26657-95-4.

More preferably, the triacylycerol is selected from tristearin, CAS number: 555-43-1 or tripalmitin, CAS number: 55-44-2.

The term "alkyl" refers to a monoradical of a saturated straight or branched hydrocarbon. Preferably, the alkyl group comprises from 6 to 23 carbon atoms, i.e., 1, 2, 3, 4, 5, 6, 7, 8, 9, 10, 11, 12, 13, 14, 15, 16, 17, 18, 19, 20, 21, 22 or 23 carbon atoms, more preferably 8 to 23 carbon atoms, such as 10 to 23 or 12 to 20 carbon atoms. Exemplary alkyl groups include, n-hexyl, isohexyl, sec-hexyl, n-heptyl, iso-heptyl, n-octyl, 2-ethyl-hexyl, n-nonyl, n-decyl, and the like.

The term "alkenyl" refers to a monoradical of an unsaturated straight or branched hydrocarbon having at least one carbon-carbon double bond. Generally, the maximal number of carbon-carbon double bonds in the alkenyl group can be equal to the integer which is calculated by dividing the number of carbon atoms in the alkenyl group by 2 and, if the number of carbon atoms in the alkenyl group is uneven, rounding the result of the division down to the next integer. For example, for an alkenyl group having 9 carbon atoms, the maximum number of carbon-carbon double bonds is 4. Preferably, the alkenyl group has 6 to 23, i.e., 1, 2, 3, or 4, carbon-carbon double bonds. Preferably, the alkenyl group comprises from 6 to 23 carbon atoms, i.e. 6, 7, 8, 9, 10, 11, 12, 13, 14, 15, 16, 17, 18, 19, 20, 21, 22 or 23 carbon atoms, more preferably 8 to 23 carbon atoms, such as 10 to 23 carbon atoms or 12 to 20 carbon atoms. Thus, in a preferred embodiment, the alkenyl group comprises from 6 to 23 carbon atoms and 1, 2, 3, 4, 5, 6, 7, 8, 9, 10, or 11 carbon-carbon double bonds, more preferably it comprises 10 to 23 carbon atoms and 1, 2, 3, 4, 5, 6, 7, 8, 9, 10, or 11 carbon-carbon double bonds, such as 12 to 20 carbon atoms and 1, 2, 3, 4, 5, 6, 7, 8, 9, 10 carbon-carbon double bonds may be in cis (Z) or trans (E) configuration. Exemplary 1-hexenyl, 2-hexenyl, 3-hexenyl, 4-hexenyl, 5-hexenyl, 1-heptenyl, 2-heptenyl, 3-heptenyl, 4-heptenyl, 5-heptenyl, 6-heptenyl, 1-octenyl, 2-octenyl, 3-octenyl, 4-octenyl, 5-octenyl, 6-octenyl, 7-octenyl, 1-nonenyl, 2-nonenyl, 3-nonenyl, 4-nonenyl, 5-nonenyl, 6-nonenyl, 7-nonenyl, 8-nonenyl, 1-decenyl, 2-decenyl, 3-decenyl, 4-decenyl, 5-decenyl, 6-decenyl, 7-decenyl, 8-decenyl, 9-decenyl, and the like. If an alkenyl group is attached to a nitrogen atom, the double bond cannot be alpha to the nitrogen atom.

Preferably, the second layer comprises of from 0.1 to 60%, more preferably 1 to 50%, most preferably 10 to 25%, based on 100% by weight of the second layer. Preferably, the antibiotic is selected from a group consisting of beta-lactam antibiotics (β-lactams), penicillins, cephalosporins, monobactams, carbapenems, aminoglycoside antibiotics (aminoglycosides), quinolone antibiotics (quinolones), Glycopeptide antibiotics (glycopeptides), Lincosamide antibiotics (lincosamides), Macrolide antibiotics (macrolides), Ketolide antibiotics (ketolides), Nitroimidazole derivatives (nitroimidazoles), Polypeptide antibiotics (polypeptides), Sulfonamide antibiotics (sulfonamides), Diaminopyrimidines (trimethoprim), Tetracycline antibiotics (tetracyclines), Oxazolidinone antibiotics (oxazolidinones), lipopeptide antibiotics (lipopeptides), others such as rifampicin, chloramphenicol, tigecycline, mupirocin, fosfomycin and other anti-infective, antibiotic bactericidal, bacteriostatic substances more preferably vancomycin, daptomycin, rifampicin, fosfomycin, gentamycin, chloramphenicol, tigecyclin, mupirocin and any combination thereof.

The second layer may further comprise a saccharide, preferably the saccharide is at least one of maltotetraose, maltotriose, maltodextrine or a combination thereof. Preferably, the second layer comprises of from 0.1 to 10% by weight of the saccharide, based on 100% by weight of the second layer.

The second layer may further comprise phages. Preferably, the second layer comprises phages-enzymes, more preferably the phage-enzymes are endolysins, most preferably the endolysins are Staphefekt^{™} (Micreos, 3721 MA Bilthoven, Netherlands).

If phages-enzymes are comprised, a targeted therapy addressing at least one bacteria species would be possible. In this respect it is referenced to: Hamed Haddad Kashani, Mathias Schmelcher, et al. (20217) - Recombinant Endolysins as Potential Therapeutics against Antibiotic-Resistant Staphylococcus aureus: Current Status of Research and Novel Delivery Strategies Clin Microbiol Rev. 2017 Nov 29;31(1):e00071-17. doi: 10.1128/CMR.00071-17 and Ayesha Lone, Hany Anany, et al. (2016) Development of prototypes of bioactive packaging materials based on immobilized bacteriophages for control of growth of bacterial pathogens in foods; Int J Food Microbiol 2016 Jan 18;217:49-58, doi: 10.1016/j.ijfoodmicro.2015.10.011.Epub 2015 Oct 22.

Preferably, the second layer comprises of from 0.01 to 20%, more preferably 0.1 to 15%, most preferably 0.5 to 10%, and particularly preferred 1.5 to 10%, particularly preferred 5 to 10 %, by weight phage-enzymes, based on 100% by weight of the second layer.

Preferably, the second layer has a thickness of from 1 to 50 µm.

Preferably, the second layer in the implanted implant is biologically decomposed after at least 3 weeks, more preferably after at least 6 weeks, most preferably after at least 12 weeks.

The coating may further comprise a third layer arranged on the second layer, wherein the third layer comprises at least one lipid and at least one antibiotic and/or silver ions.

Preferably, the at least one lipid is a monoacylglycerol, diacyglycerol, triacylglycerol, sterol, saturated and/or unsaturated fatty acid, saturated and/or unsaturated fatty acid-salts, and/or saturated fatty alcohol, and/or saturated fatty acid ester and/or a combination thereof. Wherein the monoacylglycerol, diacyglycerol, triacylglycerol, sterol, saturated and/or unsaturated fatty acid, saturated and/or unsaturated fatty acid-salts, and/or saturated fatty alcohol, and/or saturated fatty acid ester is defined as above for the second layer.

Preferably, the third layer comprises of from 0.1 to 60%, more preferably 1 to 50%, most preferably 10 to 25%, by weight of the antibiotic, based on 100% by weight of the second layer. Preferably, the antibiotic is selected from a group consisting of beta-lactam antibiotics (β-lactams), penicillins, cephalosporins, monobactams, carbapenems, aminoglycoside antibiotics (aminoglycosides), quinolone antibiotics (quinolones), Glycopeptide antibiotics (glycopeptides), Lincosamide antibiotics (lincosamides), Macrolide antibiotics (macrolides), Ketolide antibiotics (ketolides), Nitroimidazole derivatives (nitroimidazoles), Polypeptide antibiotics (polypeptides), Sulfonamide antibiotics (sulfonamides), Diaminopyrimidines (trimethoprim), Tetracycline antibiotics (tetracyclines), Oxazolidinone antibiotics (oxazolidinones), lipopeptide antibiotics (lipopeptides), others such as rifampicin, chloramphenicol, tigecycline, mupirocin, fosfomycin and other anti-infective, antibiotic bactericidal, bacteriostatic substances more preferably vancomycin, daptomycin, rifampicin, fosfomycin, gentamycin, chloramphenicol, tigecyclin, mupirocin and any combination thereof.

The invention is further directed to a method of manufacturing an implant. Preferably, an implant as described above. The method comprises forming a first layer arranged on at least a portion of a surface of the implant, said forming of the first layer comprising contacting at a least a portion of the surface of the implant with a first coating composition comprising at least one polylactide and silver ions.

The implant and/or the surface of the implant may be made of any material suitable for implants. The implant and/or the surface of the implant may be made made of a metal or a non-metal such as a polymer.

Preferably, the implant and/or the surface is made of metal. Preferably, the implant and/or the surface material comprise 316 LVM implant steel, Co-Cr-Mo, TiN coated Cr-Co-Mo, titanium or a titanium alloy. Preferably, the titanium alloy is Ti₆Al₄V.

In one embodiment, at least the surface of the implant is made of metal. Preferably, the surface material comprises metal such as 316 LVM implant steel, Co-Cr-Mo, TiN, coated Cr-Co-Mo, titanium or a titanium alloy. Preferably, the titanium alloy is Ti₆Al₄V.

In one embodiment, at least the portion of surface of the implant, which is coated, is made of metal. Preferably, the surface material comprises a metal such as 316 LVM implant steel, Co-Cr-Mo, TiN coated Cr-Co-Mo, titanium or a titanium alloy. Preferably, the titanium alloy is Ti₆Al₄V.

In one embodiment, at least the surface of the implant is made of polymer. Preferably, the implant and/or the surface material comprises a polymer consisting of the group polyether ether ketone (PEEK), ultra-High molecular weight polyethylene (UHMWPE), low density polyethylene (LDPE), polymethylmethacrylate (PMMA), or polyphenylsulfone (PPSU). More preferably, the polymer is PEEK.

Preferably, the implant and/or the surface of the implant comprises at least 30 wt.-%, more preferably at least 60 wt.-%, most preferably at least 80 wt.-%, particular preferred at least 90 wt.-%, most particular preferred at least 95 wt.-% of a polymer based on the overall weight of the implant.

Preferably, the implant and/or the surface of the implant comprises less than 30 wt.-%, more preferably less than 60 wt.-%, most preferably less than 80 wt.-%, particular preferred less than 90 wt.-%, most particular preferred less than 95 wt.-% of a polymer based on the overall weight of the implant.

In one embodiment, at least the portion of surface of the implant which is coated is made of a polymer. Preferably, the surface material comprises PEEK. More preferably, the polymer is PEEK.

A silver metal layer may be arranged between the surface of the implant and the first layer.

A calcium oxalate layer, may be arranged between the surface of the implant and the first layer.

The surface of the implant to be coated may be treated with glass bead blasting.

If the implant and/or the surface material is made of metal, preferably titanium or a titanium alloy, the implant and/or the surface material may be pretreated and/or etched, preferably to achieve osteointegration/osteoinduction with at least with one of the following hydrochloric acid, sulfuric acid, hydrogen peroxide, hydrofluoric acid, nitric acid, oxalic acid, potassium hydroxide, calcium lactate and dihydrogen phosphate. In this respect it is referred to Souza, Julio C. M.; Sordi, Mariane B.; Kanazawa, Miya; Ravindran, Sriram; Henriques, Bruno; Silva, Filipe S. et al. (2019): Nano-scale modification of titanium implant surfaces to enhance osseointegration. In: Acta Biomaterialia 94, S. 112-131. DOI: 10.1016/j.actbio.2019.05.045.

Preferably, the first layer comprises at least one polylactide selected from the group consisting of poly(L-lactide) (CAS number: 33135-50-1), poly(D-lactide), poly(L-lactide-co-D,L-lactide) (CAS number: 52305-30-3), poly(D-lactide-co-glycolide), poly(L-lactide-co-glycolide) (CAS number: 30846-39-0), poly(L-lactide-co-caprolactone) (CAS number: 65408-67-5), and Poly(L-lactide-co-trimethylene carbonate) (CAS number: 113883-70-8), poly(D,L-lactide-co-glycolide) (CAS number: 1354955-03-5), more preferably poly(L-lactide).

Preferably, the first layer comprises at least 70%, more preferably at least 80%, most preferably at least 90%, particularly preferred at least 95%, more particularly preferred at least 99% of the at least one polylactide.

Preferably, the silver ions are provided in form of a silver salt or a silver complex compound. More preferably, the silver ions are provided in form of a silver salt. Most preferably, the silver salt is selected from the group consisting of silver sulfadiazine, silver diethyldithiocarbamate, silver oxide, silver carbonate and silver nitrate, silver acetate, silver benzoate, silver iodate, silver laurate, silver protein, silver chloride, silver palmitate or combination thereof. Even more preferred silver diethyldithiocarbamate, particularly preferred silver nitrate.

Preferably, the first coating composition may comprise hydroxyapatite, or another poorly soluble calcium compound e.g. calcium carbonate.

Preferably, the first coating composition may comprise endolysins.

The first coating composition comprises solvent, which is pyridine. The solvent can be completely removed by heating in the manufacturing process.

Preferably, at least a portion of the surface of the implant is contacted with the first coating composition by dip coating or spray coating.

Preferably, forming of the first layer comprises drying.

The method may further comprise forming a second layer arranged on the first layer, said forming of the second layer comprising contacting the first layer with a second coating composition comprising at least one poly(lactide-co-glycolide) and at least one antibiotic/antiinfective and/or silver ions.

Preferably, the poly(lactide-co-glycolide) in the second layer is poly(D-lactide-co-glycolide), or poly(L-lactide-co-glycolide) (CAS number: 30846-39-0), or poly(D,L-lactide-co-glycolide) (CAS number: 1354955-03-5).

Preferably, the antibiotic is selected from a group consisting of beta-lactam antibiotics (β-lactams), penicillins, cephalosporins, monobactams, carbapenems, aminoglycoside antibiotics (aminoglycosides), quinolone antibiotics (quinolones), Glycopeptide antibiotics (glycopeptides), Lincosamide antibiotics (lincosamides), Macrolide antibiotics (macrolides), Ketolide antibiotics (ketolides), Nitroimidazole derivatives (nitroimidazoles), Polypeptide antibiotics (polypeptides), Sulfonamide antibiotics (sulfonamides), Diaminopyrimidines (trimethoprim), Tetracycline antibiotics (tetracyclines), Oxazolidinone antibiotics (oxazolidinones), lipopeptide antibiotics (lipopeptides), others such as rifampicin, chloramphenicol, tigecycline, mupirocin, fosfomycin and other anti-infective, antibiotic bactericidal, bacteriostatic substances more preferrably vancomycin, daptomycin, rifampicin, fosfomycin, gentamycin, chloramphenicol, tigecyclin, mupirocin and any combination thereof.

The second coating composition may further comprise a saccharide. Preferably, the saccharide is at least one of maltotetraose, maltotriose, maltodextrine or combinations thereof.

Preferably, the second coating composition may comprise endolysins.

Preferably, the second coating composition comprises a solvent for poly(lactide-co-glycolide) (e.g. acetone, dimethyl sulfoxide, formic acid) and for lipid (e.g. pentane, heptane). More preferably for poly(lactide-co-glycolide), the solvent is dimethyl sulfoxide, for TAG is pentane and cholesterol is tert-butyl methyl ether. The solvent can be completely removed by heating in the manufacturing process.

The first layer may be contacted with the second coating composition by dip coating or spray coating.

Preferably, forming of the second layer further comprises drying.

The method may further comprise forming a second layer arranged on the first layer, said forming of the second layer comprising contacting the first layer with a second coating composition comprising at least one lipid and/or at least one polylactide and at least one antibiotic and/or at least one silver salt.

In a further embodiment, the method comprises forming a first layer arranged on at least a portion of a surface of the implant, said forming of the first layer comprising contacting at a least a portion of the surface of the implant with a coating composition comprising at least one poly(lactide-co-glycolide) and/or at least one lipid and at least one antiinfective/antibiotic, preferably a coanting composition as referred to above as "second coating composition". This means that the second coating composition is arranged directly on the implant's surface in this embodiment forming the first layer instead of the first coating composition.

Preferably, the at least one lipid is a monoacylglycerol, diacyglycerol, triacylglycerol, sterol, saturated and/or unsaturated fatty acid, saturated and/or unsaturated fatty acid-salts, and/or saturated fatty alcohol, and/or saturated fatty acid ester and/or a combination thereof as further defined above for the first layer. More preferably, the at least one lipid is a triacyglycerol.

Preferably, the antibiotic is selected from a group consisting of beta-lactam antibiotics (β-lactams), penicillins, cephalosporins, monobactams, carbapenems, aminoglycoside antibiotics (aminoglycosides), quinolone antibiotics (quinolones), Glycopeptide antibiotics (glycopeptides), Lincosamide antibiotics (lincosamides), Macrolide antibiotics (macrolides), Ketolide antibiotics (ketolides), Nitroimidazole derivatives (nitroimidazoles), Polypeptide antibiotics (polypeptides), Sulfonamide antibiotics (sulfonamides), Diaminopyrimidines (trimethoprim), Tetracycline antibiotics (tetracyclines), Oxazolidinone antibiotics (oxazolidinones), lipopeptide antibiotics (lipopeptides), others such as rifampicin, chloramphenicol, tigecycline, mupirocin, fosfomycin and other anti-infective, antibiotic bactericidal, bacteriostatic substances more preferably vancomycin, daptomycin, rifampicin, fosfomycin, gentamycin, chloramphenicol, tigecyclin, mupirocin, and any combination thereof.

The second coating composition may further comprise a saccharide. Preferably, the saccharide is at least one of maltotetraose, maltotriose, maltodextrine or combinations thereof.

Preferably, the second coating composition may comprise endolysins.

Preferably, the second coating composition may comprise a solvent, more preferably the solvent is pentane.

The first layer may be contacted with the second coating composition by dip coating or spray coating.

Preferably, forming of the second layer further comprises drying.

The method may further comprise forming a third layer arranged on the second layer, said forming of the third layer comprising contacting the second layer with a second coating composition comprising at least one lipid and/or at least one polylactide and at least one antiinfective/antibiotic and/or a silver salt.

Preferably, the at least one lipid is a monoacylglycerol, diacyglycerol, triacylglycerol,, sterol, saturated and/or unsaturated fatty acid, saturated and/or unsaturated fatty acid-salts, and/or saturated fatty alcohol, and/or saturated fatty acid ester and/or a combination thereof and as further defined above for the first layer. More preferably, the at least one lipid is a triacyglycerol.

Preferably, the antibiotic is selected from a group consisting of beta-lactam antibiotics (β-lactams), penicillins, cephalosporins, monobactams, carbapenems, aminoglycoside antibiotics (aminoglycosides), quinolone antibiotics (quinolones), Glycopeptide antibiotics (glycopeptides), Lincosamide antibiotics (lincosamides), Macrolide antibiotics (macrolides), Ketolide antibiotics (ketolides), Nitroimidazole derivatives (nitroimidazoles), Polypeptide antibiotics (polypeptides), Sulfonamide antibiotics (sulfonamides), Diaminopyrimidines (trimethoprim), Tetracycline antibiotics (tetracyclines), Oxazolidinone antibiotics (oxazolidinones), lipopeptide antibiotics (lipopeptides), others such as rifampicin, chloramphenicol, tigecycline, mupirocin, fosfomycin and other anti-infective, antibiotic bactericidal, bacteriostatic substances more preferably vancomycin, daptomycin, rifampicin, fosfomycin, gentamycin, chloramphenicol, tigecyclin, mupirocin, and any combination thereof.

The third coating composition may further comprise a saccharide. Preferably, the saccharide is at least one of maltotetraose, maltotriose, maltodextrine or combinations thereof.

Preferably, the third coating composition may comprise endolysins.

Preferably, the third coating composition comprises a solvent for polylactide (e.g. dimethyl sulfoxide, formic acid) and for lipid (e.g. pentane, heptane). More preferably for polylactide, the solvent is dimethyl sulfoxide, for TAG is pentane and cholesterol is tert-butyl methyl ether. The solvent can be completely removed by heating in the manufacturing process.

The second layer may be contacted with the third coating composition by dip coating or spray coating.

Preferably, forming of the third layer further comprises drying.

The invention is further directed to an implant, obtainable by a method as described above.

The invention is further directed to a coating of an implant as defined above for use in a method for preventing or treating a local infection with a pathogen near the implanted implant exhibiting said coating as defined above, wherein the method comprises exposure of the first layer of the coating of the implanted implant to shock waves resulting in an increased release of silver ions from the first layer of the coating.

Wherein local infection means that the effect of the released silver anions is local in the subject and not systemic.

Silver ions exhibit a broad-spectrum activity against both gram-positive and gram-negative bacteria, fungi, protozoans, and viruses and are less likely to induce bacterial resistance than antibiotics (Brennan et al. 2015, Chaloupka et al. 2010).

Wherein the pathogen's growth may be inhibited or killed by silver anions. Preferably the pathogen is selected from bacteria and fungi, protozoans and viruses, more preferably from bacteria.

Preferably, the bacteria are gram-positive or gram-negative bacteria, more preferably selected from the group consisting of staphylococcus aureus, coagulase negative staphylococcus, streptococcus, enterococcus, anaerobic bacteria.

Preferably, the fungi is *Candida albicans.*

Acoustic shock waves are commonly applied in medicine for the treatment of several indications. Thus, the person skilled in the art may use commercially available equipment and established procedures to apply the shock waves to expose the implanted implant to the shock waves in order to induce the increased release of silver ions from the first layer.

The shock waves are acoustic waves with a pressure gradient of 1 to 1500 MPa/mm.

In one embodiment, the shock waves exhibit at least one of the following characteristics a) - h):
a. the peak pressure is 10 to 150 M Pa:
b. the pressure gradient is 10 to 150 MPa/mm
c. the impulse duration is 10 to 10000 ns, more preferably 100 to 1000 ns, more preferably 150 to 500 ns;
d. the shock wave transmission into the tissue is 0.1 mm to 1 m, more preferably 1 mm to 500 mm, most preferably 10 to 200 mm;
e. the energy of one shock wave is 0.1 to 100 mJ, more preferably 1 to 50 mJ, most preferably 10 to 40 mJ;
f. the positive pressure is major to the negative pressure component of one shock wave;
g. the shock wave transfers an energy which is sufficient to overcome the tensile strength of the coating material of the first layer to the surface of the implant, resulting in removal of at least a part of the first layer from the implant, wherein the tensile strength is 5 to 70 MPa, preferably 10-60 MPa;
h. the energy flux density of the shock waves is 0.1 to 20 mJ/mm², preferably 0.5 to 10 mJ/mm², more preferably 1 to 5 mJ/mm², most preferably 1.1 to 3 mJ/mm². Wherein the energy flux density corresponds to the energy transferred / surface area.

A better understanding of the present invention and of its advantages will be had from the following examples, offered for illustrative purposes only.

### EXAMPLES OF THE INVENTION

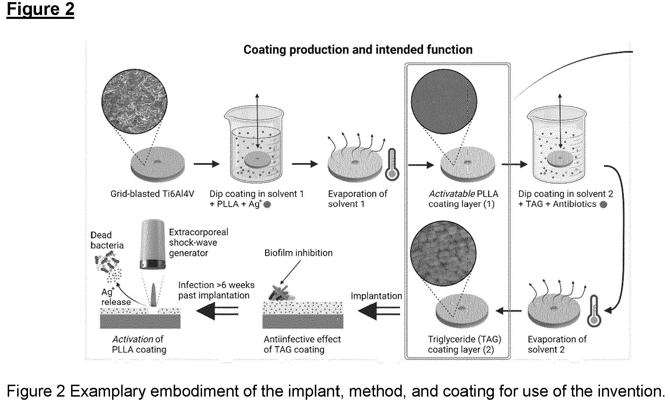

An overview of a coating variant with activation is summarized in a graphical abstract figure 2.

### 1 Coating methods

### 1.1 First layer coating

A durable layer (approx. 10 µm) of poly-L-lactide (Resomer ^{®} L 206 S, Evonik Health Care, Darmstadt) with a soluble silver compound is applied to the implant made of a frequently used titanium alloy (Ti₆Al₄V Grade 5). Resomer L 206 S is a very slowly (>10 years) absorbable biocompatible layer that releases homogeneously dissolved silver ions when colonized with bacteria. Three different processes have been developed, from which result different silver release.

### Silver coatings:

Variant 1: 100 mg Resomer L206 S are dissolved in 900 µL 0.5% Ag-diethyldithiocarbamate in chloroform in a 3 mL PFA (perfluoroalkoxy-polymer) vessel. The implant specimen is briefly immersed and dried hanging under an S2 workbench for 1 hour at room temperature. Ag-diethyldithiocarbamate is a nontoxic silver compound homogeneously soluble in nonpolar polymers such as L206 S or chloroform. By drying, the solvent chloroform (boiling point 61 °C) is completely removed.

Variant 2: In a 3 mL PFA vessel, mix 25 µL of 10% AgNO₃ solution (shake 100 mg AgNO₃ (powdered) + 1 ml chloroform + 100 µl pyridine at room temperature for several hours until crystals are dissolved) with 1 mL of 10% Resomer (100 mg) L 206 S in chloroform. The implant specimen is briefly immersed. The test specimen is then heated to 150 °C for 3 min. Silver nitrate is also soluble in organic solvents in small quantities, as a silver pyridine complex well soluble. After homogeneous solution in the polymer, the pyridine (boiling point 115 °C) is removed completely by heating to 150 °C.

Variant 3: In a 3 mL PFA vessel, mix 25 µl of 10% AgNO₃ solution (shake 100 mg AgNO₃ (powdered)+ 1 ml chloroform + 100 µl pyridine at room temperature for several hours until crystals are dissolved) with 1 mL of 10% Resomer (100mg) L 206 S in chloroform. The implant specimen is briefly immersed. The test specimen is then heated to 200° C for 1-2 min until the polymer is melted clear. The increased temperature to 200 °C promotes adhesion to the titanium substrate and the homogeneity of the surface of the layer. In the case of real implants, the amount of solution can be scaled accordingly and the PFA vessels can be replaced by glass vessels of an appropriate size (glass blowing).

### 1.2 Two layer coating

### 1.2.1 Ti₆Al₄V Substrate

Typical metallic biomaterials in medical technology are Co-Cr-Mo alloys, pure titanium and titanium alloys (Karacan et al. 2018) as well as Co-Cr-Mo alloys with surface modifications like TiN and others. Typical polymers in medical technology are PEEK, UHMWPE, LDPE, PMMA, PPSU. The selection of these materials results from the requirements regarding corrosion resistance, biocompatibility, bio-adhesion and favorable mechanical properties (Peters et al. 2002). Due to its extensive use as an implant material in orthopedics, the titanium alloy Ti₆Al₄V Grade 5 was chosen as preferred substrate material for the present coating system. The surface was prepared using glass bead blasting (*d*ₖ = 150 - 250 µm, Eisenwerk Würth GmbH, Bad Friedrichshall, Germany) in accordance with recommendations by Guo, Matinlinna et al. with a pressure of p = 2.5 bar for 12 s in a distance of 65 mm. The substrate geometry was defined as disc with a diameter of 14 mm, a thickness of 1.5 mm and a centric hole with a diameter of 2 mm.

### 1.2.2 Substrate surface modification and analysis

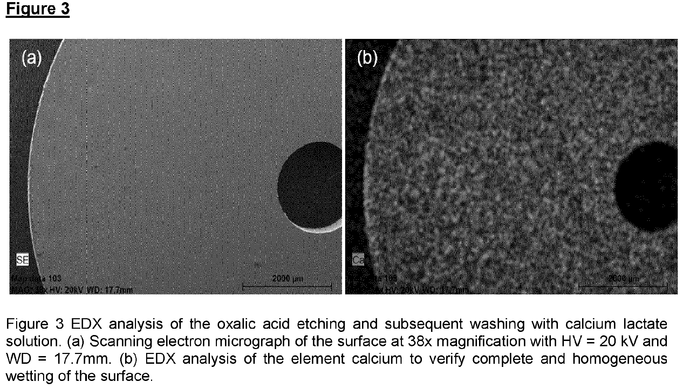

Standardized titanium alloy substrates were used for the evaluation of the coatings (Ti₆Al₄V Grade 5). To characterize the mechanical properties of the coating system on these, surface texture analysis was performed. In a first step glass bead blasting was used in accordance with data from the literature (Byrne et al. 2013; Guo et al. 2019). In a second step a novel, very promising method was conceived: A oxalic acid (H₂C₂O₄) etching followed by rinsing with 1 m calcium lactate solution results in a finer structure (Rₐ 0.25 µm) compared to glass bead blasting (Rₐ 0.67 µm). The resulting compound calcium oxalate is extremely stable and insoluble and thus resists absorption in the body. The homogeneity of this coating was validated by an energy dispersive X-ray spectroscopy (EDX-analysis) using a scanning electron microscope (SEM) and XFLASHr^{®} 6110 detector (Bruker Co., Billerica, Massachusetts, USA) (figure 3).

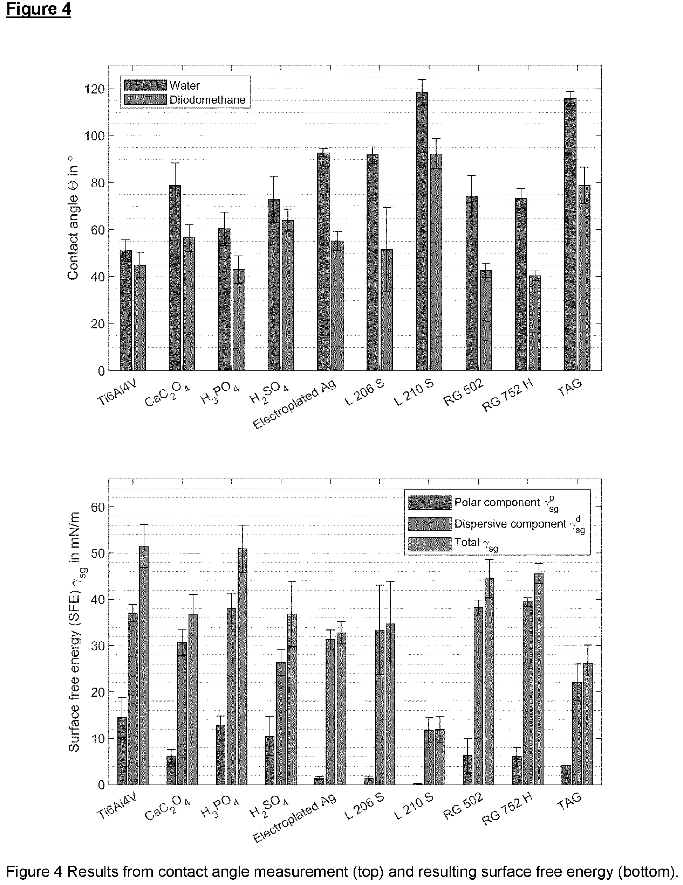

Pull-off tensile tests revealed a 2.3-fold increased bond strength between the coating and the Ti₆Al₄V substrate of 10 MPa (without) to 23 MPa with CaC₂O₄ etching. This complies with the ASTM F1147-05(2017)e1 standard requirement for coatings on medical implants of 22 MPa. Hydrophobicity and a low surface free energy comparable to silver were determined from contact angle measurement, whereas Ti6Al4V was hydrophilic with a higher surface free energy indicating a lowered risk for bacterial adhesion for the etched surface (figure 4).

### 1.2.3 Biopolymer coating -1^{st} Layer

The first layer with activatable properties consisting of a Poly(L-lactide) (PLLA - Resomer L206 S, Evonik Health Care, Darmstadt, Germany) The PLLA is soluble in chloroform, and 1 mL of 10% PLLA were loaded with 25 µL homogeneously dissolved silver ions (Ag⁺) in concentrations of 2 % / 6 % / 8 %.

All steps were performed under sterile conditions in a S2 workbench (HERAsafe HS12 - Thermo Scientific GmbH, Dreieich - Germany). The substrate was sterilized by immersion in 70 % ethanol and after evaporation manually dip-coated by immersion/emergence within 5 seconds. The substrate is then heated to 200° C for 1-2 min until the polymer is melted clear, which completely removes the solvent chloroform. After cooling sterilization with a short immersion in 70 % ethanol and subsequent evaporation was then repeated. The coated substrates were stored in sterile bags (SteriBag, 60 ml, 127 x 76 mm Bürkle GmbH, Germany) until testing at the next day. The anti-infective effect of this coating is the release of incorporated silver ions induced by an *activation* with a extracorporeal shock wave (ESW) which results in a local detachment of coating fragments.

### 1.2.4 Lipid or Biopolymer coating - 2^{nd} Layer

The second coating layer is designed for a loading with anti-infectives and for resorption within weeks to reduce the initial risk of infection after implantation on the one hand, and in case of an infection to locally support the systemic therapy on the other hand. Different lipids (triacylglycerol (TAG) tristearin CAS number: 555-43-1 and tripalmitin CAS number: 55-44-2 (Sigma-Aldrich Chemie GmbH, Deisenhofen, Germany), cholesterol (CAS number: 57-88-5, Sigma-Aldrich Chemie GmbH, Deisenhofen, Germany), diacylglycerol dipalmitin (CAS number: 26657-95-4 Sigma-Aldrich Chemie GmbH, Deisenhofen, Germany), monoacylglycerol α-monolaurate (CAS number: 142-18-7, Sigma-Aldrich Chemie GmbH, Deisenhofen, Germany)) were included. The preferred lipid coating consisted of triacylglycerol (TAG).

The TAG is applied by dipping the substrate in a 10 % triacylglycerol solution in the solvent pentane with a finely powdered (mortared and sieved) or dissolved antibiotic. Alternatively, the antibiotic was mixed in a 20% RG 502 (PLGA) (Evonik Health Care, Darmstadt, Germany) solution in dimethyl sulfoxide or formic acid alone or with 100 µl 20% lauricidin (monolaurin) in formic acid with 100 mg cholesterol in 800 µl TBME, under sonication. The solvent is removed by drying at room temperature in a hanging position and followed by sterilization with a short immersion in 70 % ethanol and subsequent evaporation. The coated substrates were stored until testing in sterile bags until next day. All steps were performed under sterile conditions in a S2 workbench. The coating degrades with defined kinetics, constantly releasing anti-infectives within the first 6 weeks and 3 months, respectively.

### 2 Release of silver ions

### 2.1 Silver determination with atomic absorption spectrometry

Samples were transferred to a 500 µL PFA vessel (AHF-Analysetechnik, Tübingen, Germany). 100 µL HNO₃ (0.16 mol/L) was added and incubated overnight at room temperature. Before measurement, the samples were incubated at 70 °C for 90 min, cooled, and diluted 10-fold with H₂O. A volume of 20 µL of the solution was added directly to the graphite furnace atomic absorption spectrometry (GF-AAS) (AAS-6300, Shimadzu, Kyoto, Japan). Triplicate measurements were performed for each determination. The recommended operating conditions for the spectrometer were: Lamp current of the hollow cathode lamp: 12 mA; absorption wavelength: 328.1 nm; BGC-D2 mode; slit width: 0.7 nm. Working standard solutions containing 0, 0.5, 1, 2, and 4 µg/L Ag (Standard Merck, Germany) were used. The intra- and inter-day precision of the assay, expressed as coefficient of variation (CV%), ranged from 2.8 to 6.5%. The recommended operating conditions for the GF-AAS nebulizer are listed in Table 1.

**Table 1: Temperature program atomic absorption spectrometry (GF-AAS)**

| Stage | T [°C] | Time [s] | Mode | Sens. | Argon flow [l/min] |
|---|---|---|---|---|---|
| 1 | 200 | 20 | RAMP | OFF | 0.1 |
| 2 | 500 | 10 | RAMP | OFF | 0.1 |
| 3 | 400 | 5 | RAMP | OFF | 1 |
| 4 | 400 | 5 | RAMP | OFF | 0 |
| 5 | 1800 | 5 | STEP | ON | 0 |
| 6 | 1800 | 2 | STEP | OFF | 0 |

Ag-Elution in water for untreated variants 1 to 3 has been determined and the results listed in Table 2.

**Table 2: Results from Ag-Elution in water after 4 days in µq/L**

| Variant | Ag |
|---|---|
| 1 | 2 |
| 2 | 400 |
| 3 | 14 |

Depending on the preparation method, only a negligible amount of silver can be eluted in recipes 1 and 3.

The prepared samples according to variant 1 to 3, as prepared above, have been treated with acoustic shock waves and Ag-elution has been determined. The results are listed in Table 3.

**Table 3: Ag-Elution in µq/L as a result of extracorporeal shock wave (ESW) treatment**

| Variant | Ag at low intensity | Ag at high intensity |
|---|---|---|
| 1 | 46 | 55 |
| 2 | 3580 | 7853 |
| 3 | 1115 | 43794 |

The silver content in the supernatant (aqua) measured by graphite furnace atomic absorption spectrometry changes depending on the coating production method and the intensity of the applied shock wave.

### 2.2 Shock wave treatment

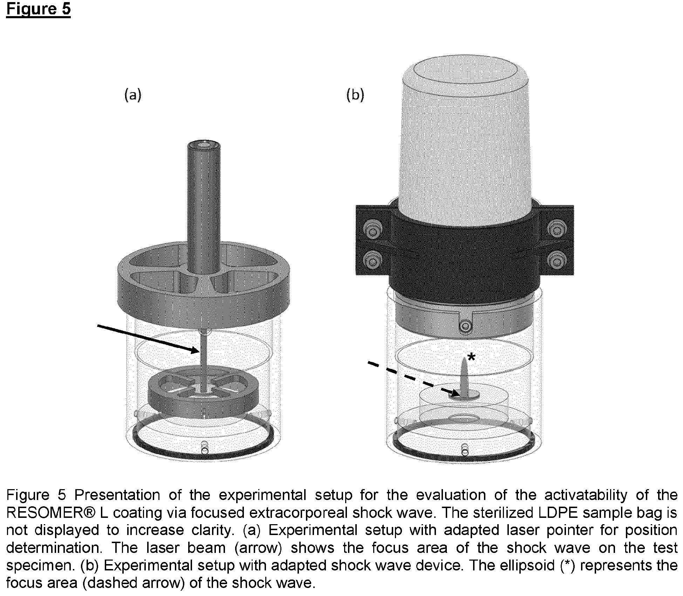

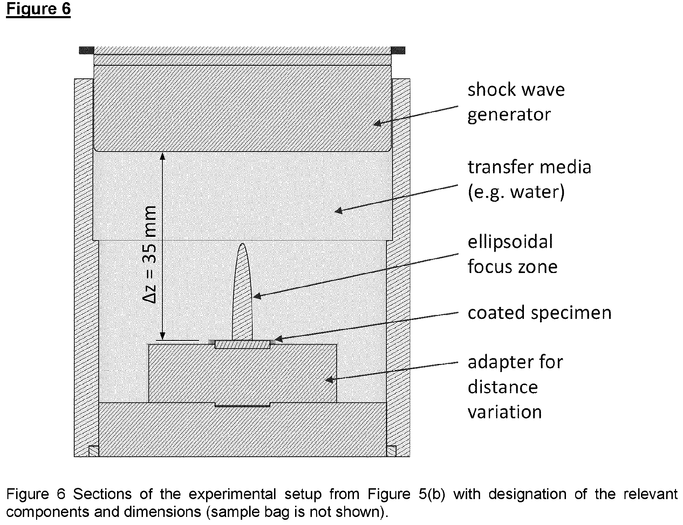

In order to be able to detect and analyze the possibility of mechanical activation of the RESOMER^{®} L coating, the test bench shown in figure 5 and figure 6 was developed. First, the sterilized coated specimens were placed in a sterilized LDPE bag (SteriBag, Bürkle GmbH, Bad Bellingen, Germany) and filled with 1 ml of water for injections (Aqua, B. Braun SE, Melsungen, Germany) using a pipette. The bag filled with liquid and test specimen was then vacuumed and hertight welded. In the next step, the filled bag was placed on the adapter and fixed by means of the hold-down holder. To control positioning, the laser pointer shown in figure 5(a) can be used and the position can be adjusted by rotating the adapter. For the effective transmission of the shock wave from the shock wave device towards the sample surface, water was chosen as the transfer medium.

Due to the comparable acoustic impedance Z of the media water (Z_{water} = 1.48 x 10E6 Ns/m³), adipose tissue (Z_{fat} = 1.33 x 10E6 Ns/m³) and muscle tissue (Z_{muscle} = 1.67 x 10E6 Ns/m³) an appropriate transferability to the ratios *in vivo* can already be ensured. Following the filling of the interior with water, the DUOLITH SD1 shock wave device with C-ACTOR handpiece (STORZ MEDICAL AG, Tägerwilen, Switzerland) was positioned at a distance of 35 mm from the sample surface. The following parameters were selected for the subsequent shock wave application:
Low intensity
   ∘ Energy flux density ED = 1.24 mJ/mm²
   ∘ Frequency *f* = 3 Hz
   ∘ Number of pulses n = 1000
   ∘ Sample quarters used = 4
   ➢ Total energy input *Eₜₒₜ =* 162.2 kJ
High Intensity
   ∘ Energy flux density ED = 1.24 mJ/mm²
   ∘ Frequency f = 3 Hz
   ∘ Number of pulses n = 2000
   ∘ Sample quarters used = 4
   ➢ Total energy input *Eₜₒₜ* = 324.4 kJ

Following the test execution, the liquid was pippet from the sample bags and again sterilely packaged in a centrifuge tube (Safe-Lock Tubes, Eppendorf AG, Hamburg) in order to examine the amount of released silver by atomic absorption spectrometry (GF-AAS) (see above). Furthermore, the specimen was used for further scanning electron microscopic analysis of the surface degradation.

### 2.3 SEM / EDX analysis of the sample surface

For the analysis of the shattering effect of the RESOMER^{®} L coating achieved by the shock wave exposure, the scanning electron microscope (SEM) including the energy dispersive X-ray spectroscopy (EDX) was used. The REM Zeiss EVO MA10 (Carl Zeiss Microscopy GmbH, Jena, Germany) was used in conjunction with the XFLASH^{®} 6110 detector (Bruker Co., Billerica, Massachusetts, USA). In order to generate an electrical conductivity of the sample surface, it was first vaporized with a gold layer. For this purpose, DC sputtering was carried out with a current of I = 10 mA and a voltage of U = 1.17 kV for a duration of 40 seconds. The SEM analysis was carried out with an acceleration voltage of EHT = 20 kV and a working distance of WD = 10 mm using an SE detector in up to 5000x magnification.

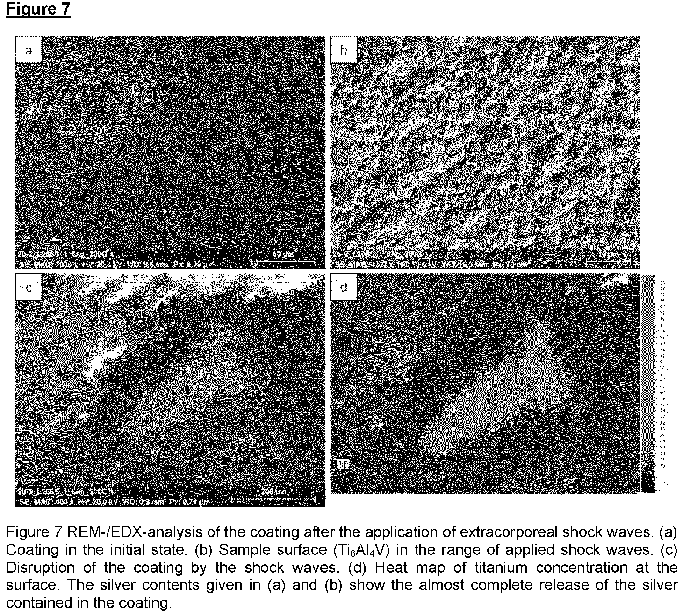

The EDX analysis was performed for both the element titanium (Ti) and the element silver (Ag). In addition to the qualitative statement via SEM, the measurement of the Ti content at the surface allows a quantitative statement about the area of the coating shattered by the shock wave treatment. The measurement of Ag content on the surface also provides quantitative information on the release of silver by measuring an area without fhESWT (1.54% Ag before shock wave) and an area after fhESWT (0.17% Ag after shock wave) of the sample surface (figure 7). Thus, the effect resulting from the shock wave treatment can be analyzed holistically.

### 3 Elution of active pharmaceutical ingredients from the biopolymer and lipid layers and microbiological/cell toxicity testing

Based on the 6-week to 3-month clinical anti-infective therapy period, the drug concentration in the eluate is quantified and the microbiological activity of the fractions is tested in a continuous flow chamber assay.

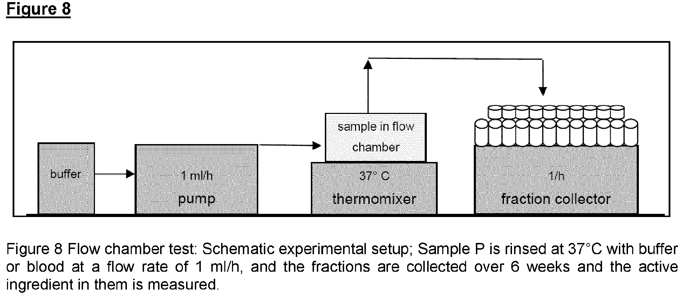

The release of the antibiotic from the layer of the test specimen (P) was studied over 6 weeks using a continuous flow chamber experiment (figure 8).

In the flow chamber (AKTA Ettan LC FPLC System, Amersham Pharmacia Biotech, Uppsala, Sweden), sample body is continuously passed through the sample chamber by a buffer liquid (PBS buffer) at a rate of 1 mL/h at 37°C using a fast protein liquid chromatography (FPLC) pump. The eluate with PBS buffer is collected once an hour using a fraction collector (Fraction Collector FRAC-950, Pharmacia, Uppsala, Sweden). Using a Kontron Uvikon photometer (Kontron, Neufahrn, Germany), the concentration of rifampin in 500 µL of the samples was quantified at 237 nm (EmM (max absorbance, PBS, pH 7.38: 33.20) and the other part was used for microbiological activity determination.

The aim is to obtain the best possible linear effective level above the minimum inhibitor concentration (MIC), and a subsequent adequate drop in the effective level after 6 weeks.

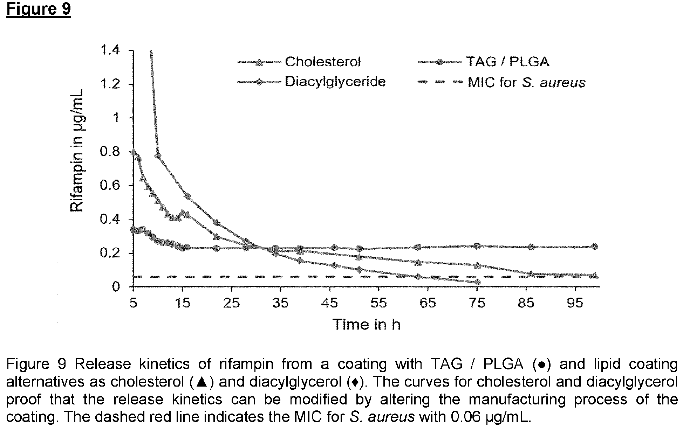

The release of active ingredients as antibiotics, as rifampin, incorporated in the lipid coating is shown in figure 9. The curve demonstrates the linear release kinetics above the level of MIC*_{S,aureus}*, i.e. a constant release of rifampin over time, without any burst effect at the beginning. This constant linear release is important since diverse studies (Ständert et al. 2021, Ren et al. 2021, Rahnamaee et al. 2021) have reported that subinhibitory concentrations (< MIC) of certain antibiotics rather enhance than inhibit biofilm formation by bacteria and can act promoting the emergence of new resistant clinical strains. The linear release of the incorporated active ingredients is in contrast to the behavior of currently used bone cement, which only releases antibiotics in a thin layer of the surface (Kuehn et al. 2005). Furthermore, the results for Lipid 2 and Lipid 3 indicate that the release kinetics can be modified.

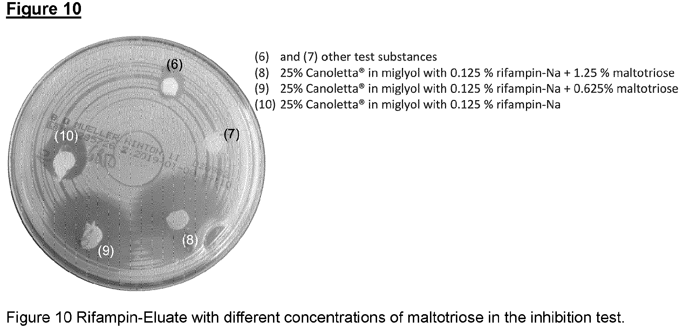

The rifampin-eluates were also tested from a series with different concentrations of maltotriose in an inhibition test shown in figure 10.

### 4 Methods for cultivation of cells

Cell cultivation is required to assess cell viability in direct contact with the coating, as well as by indirect contact with eluates or components dissolved after shock wave application.

Human dermal fibroblasts from healthy individuals were obtained from biopsies proliferated in primary cultures and then sub cultured. They are non-specific connective tissue cells with an irregular shape, whose function is to generate components of the extracellular matrix. Their isolation and proliferation in cultures is uncomplicated.

SaOs-2 cells: Osteosarcoma cells, "osteoblast-like-cells," are immortalized bone cells and are highly favored for cell culture methods because they can be easily and rapidly cultured and are free of transformation due to their sarcomatous origin as a cell line.

Human osteoblasts and fibroblasts were cultured at 37 °C in a 5% CO₂-95% atmospheric incubator. As growth medium, DMEM (Dulbecco's modification of Eagle's medium) with an addition of 10% (v/v) fetal calf serum, 2 mM L-glutamine and 1% antibiotic-antimycotic solution was used. When this medium was used, the culture wells (75 cm²) were filled with 1 ml of sterile 0.1% fibronectin solution (0.1% fibronectin in PBS) and the solution was dispersed homogeneously on the bottom of the well. After 2 h incubation at 37 °C, the cells could be seeded.

Two buffer systems were used to preserve the pH range of 7.4: HEPES (N 2-hydroxyethylpiperazine-N-2-ethanesulfonic acid, 20 mM) and sodium hydrogen carbonate (3.7 g/l). Changes in pH could be detected by the change in color of phenol red.

Cells grew as monolayer culture in tissue culture flasks (75 cm², 25 cm²), or multidishes with six wells (each 10cm²).

The intervals of medium replacement have varied with the different used cells. A medium change was required as soon as a pH decrease was indicated by the color indicator. In addition, for thawed or subcultured cells, a medium change was routinely performed the following day to remove unattached cells or cell debris.

All solutions used for subcultivation were warmed to 37 °C. The medium was removed from culture flasks (75 cm²) with confluent cell lawn for passaging. The monolayer was then briefly rinsed with 10 mL PBS/0.1% EDTA to remove calcium, magnesium (essential cations for cell adhesion) and traces of the medium. The latter may interfere with the effect of subsequent trypsin treatment and significantly prolong the time of exposure to the cells. After addition of 2 mL of trypsin (0.05%)-EDTA (0.02%) solution, the process of detachment of cells was observed under the light microscope. When the cells began to become round (approximately 1 min), the solution was aspirated, and the cells were incubated at 37 °C for 5-10 min until complete detachment. Using a pipette, the cells were rinsed with fresh medium from the bottom of the vessel, stopping the protease action of trypsin. Cells were seeded in 20 mL of medium at a dilution factor of 1:3 to 1:7, depending on requirements, and multidishes were loaded with 3 mL of medium. The amount of wash and trypsin-containing solutions could be varied depending on the contamination and size of the culture vessels.

Cell storage was performed in liquid nitrogen (-196 °C), thus guaranteeing storage for extended periods without loss of viability. It was used to store less frequently used cell lines or excess cell quantities, to protect them from variability due to repeated subculturing and from microbial contamination.

Cells from one culture flask (75 cm²) were suspended in 1 mL of medium and placed in specifically designed vessels ("cryotubes"), resulting in a concentration of approximately 1 x 10⁷ cells per ml of medium. After addition of 100 µL glycerol, the cryotubes were sealed tightly and mixed briefly until phase separation disappeared. Then the cells were cooled for 1 h in an ice bath. The tubes were then placed in a Styrofoam container and stored in the freezer at -80 °C overnight. They were then transferred to a liquid nitrogen container. To thaw the cells, the cryotubes were warmed to 37 °C in a water bath and immediately after the solid phase disappeared, they were placed in a new culture flask (75 cm²) filled with culture medium.

### 5 Cell count analysis

Analysis of viability and cytotoxicity by determining the cell count: An electronic counter (CASY^{®} Cell Counter 1, Schärfer System GmbH, Germany) was used to determine the cell count. This is a semi-automatic cell counter that works according to the resistance measuring principle. The voltage applied between two large-area platinum electrodes experiences an electrical resistance change when a cell is located between the electrodes and triggers an electrical pulse. The number of electrical pulses is equal to the number of cells. The size distribution of the cells can be recorded and evaluated by means of a measuring amplifier based on a proportional relationship to the pulse strength. Larger pulses represent vital cells, the smaller pulses represent cell debris or dead cells. Avital cells cause a smaller pulse because their cell membrane is more conductive. By shifting the cell boundaries these could be excluded from the vital cell count. For this purpose, a portion of the cell suspension was added to 10 mL of physiological saline (0.9 %, pH 7.4), the dilution factor was included, and the solution was measured three times.

Additionally, cell number was determined using a hemocytometer (Neubauer counting chamber). To distinguish between dead and living cells, vital staining with trypan blue was performed. For this purpose, after the trypsinization procedure, 0.1 mL of the cell suspension was taken up in 3.6 ml PBS and 2.7 mL of a pre-warmed 0.5 % trypan blue solution was added. The mixture was mixed and incubated at 37 °C for 2 min. In contrast to living cells, the dead cells took up the dye. This process was followed under the light microscope. The cell numbers from the two measurement methods were compared and agreed.

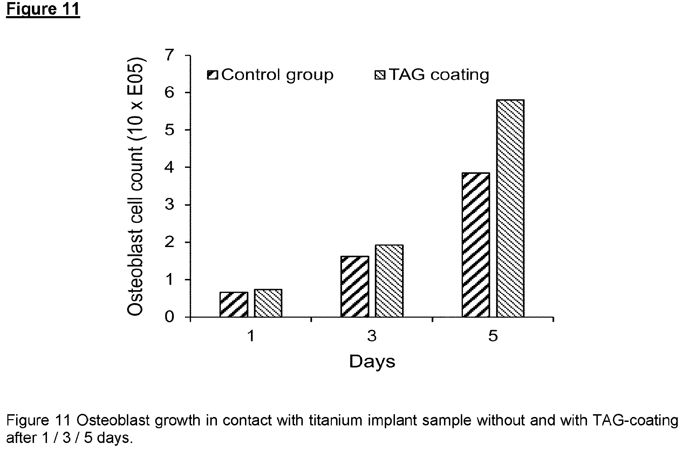

Cell number determination by the above method of osteoblasts in contact with lipid compounds (such as triacylglycerol - TAG) was performed after one, three and five days (figure 11). This shows that lipids, as used in other layers of the coating, have no negative influence on cell growth.

### 6 Determination of antibiotic susceptibility by means of the inhibition host test

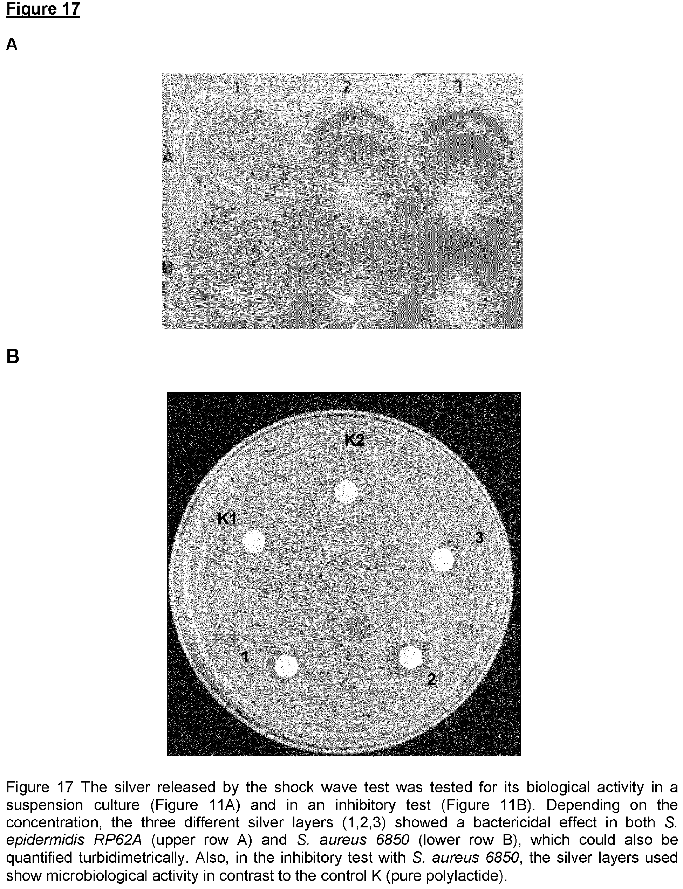

The aim of this experiment is to determine the sensitivity of cultures of S. *aureus* to supernatants containing silver ions after shock wave application (3 Hz, n = 1000, 1.24 mJ/mm²) to the polylactide coating (Resomer L206 S). For this purpose, the inhibition host test, also called agar diffusion test, was performed following EUCAST (European Committee for Antimicrobial Susceptibility Testing). On the agar plate, 2 mL of the bacterial suspension *S. aureus 6850* was added and inoculated by carefully circling the plate on the laboratory bench, distributed over the entire agar surface. The supernatant of the bacterial suspension was removed with the pipette and discarded. Test slides are placed on the agar plate and gently pressed. 100 µL of the supernatants after shock wave application are added to the test leaflets. The agar plate was incubated with the bottom side up overnight at 37 °C. Test plates inoculated with supernatants of the coating (L206 S) according to recipe 1 to 3 showed an inhibition zone and thus bactericidal effect as shown in figure 17B.

### 7 Suspension culture and bactericidal activity

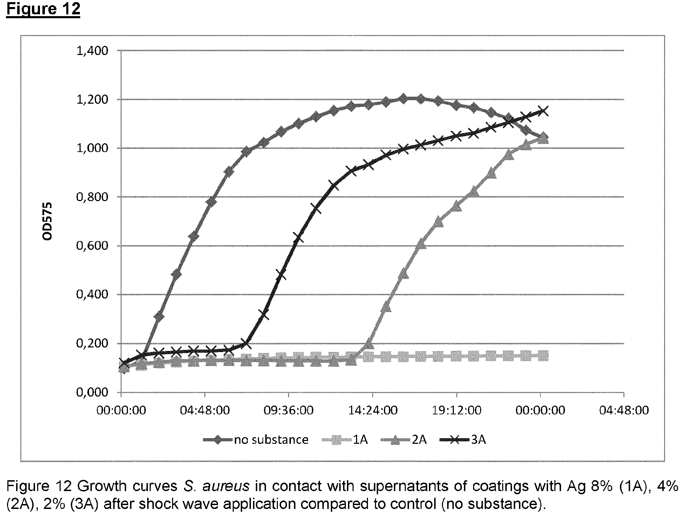

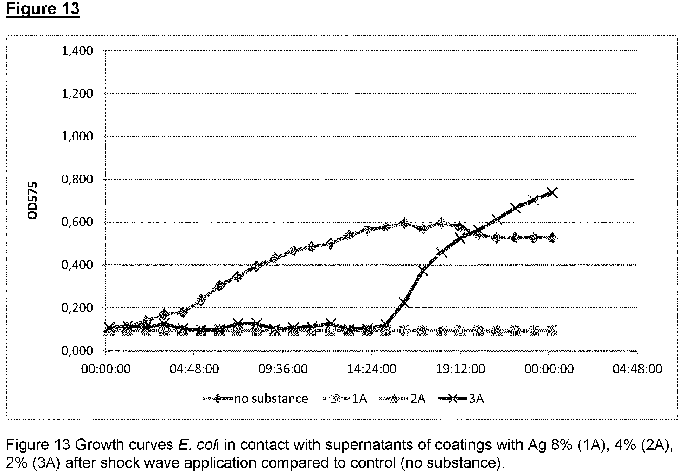

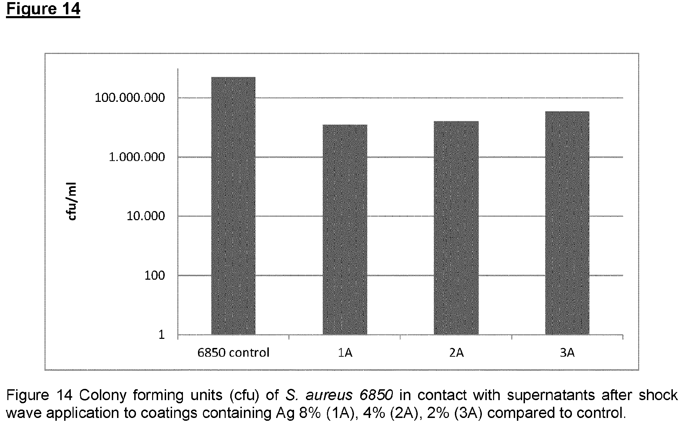

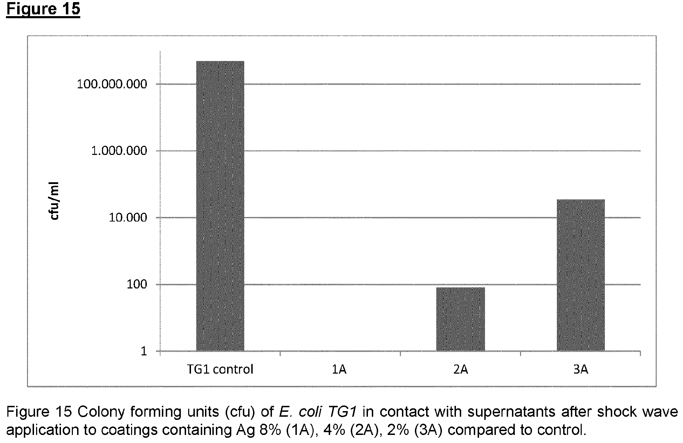

To analyze the effect of released silver from the coating (Recipe 3) after shock wave application (3 Hz, n = 1000, 1.24 mJ/mm²), supernatants were transferred to suspension cultures. Bacteria (*Staphylococcus aureus 680, Escherichia coli TG1*) from an overnight culture in Mueller Hinton II- medium (a nutrient-poor growth medium) were adjusted to approximately an optical density (OD) 600 nm of 0.1 and then pipetted 100 µL per well into a 96 well plate. In addition to an untreated control, 10 µL of sample (supernatant after shock wave/eluate solution) was added to each well. The plate was incubated at 37°C. OD was determined hourly for 24 hours in an automated manner and transferred to growth curves. Growth or inhibition of growth can be determined from an increase or decrease in OD (figure 12) and (figure 13). After 3 hours of growth, the bacteria were also plated out to check for remaining viable bacteria. The results are shown in figure 14 for *Staph. aureus,* and in figure 15 for *E. coli.*

### 8 Cell proliferation measurement

For the assessment of possible cytotoxicity, the study of cell proliferation of fibroblasts in contact with the supernatants was performed after shock wave application (3 Hz, n = 1000, 1.24 mJ/mm²) of the coating (L206 S, according to recipe 3) loaded with different concentrations of silver. Cell proliferation was determined by WST-1 assay:

A stable tetrazolium salt (WST-1) can be cleaved into a soluble formazan by a bio-reductive cellular mechanism that occurs on the surface of the cell membrane. This depends on the glycolytic formation of NAD(P)H in viable cells. The amount of formazan dye formed correlates directly with the number of metabolically active cells in the cell culture. Cells grown in a 6-well tissue culture plate were incubated with WST-1 reagent for 4 hours. For this purpose, 100 µL/well of WST-1 cell proliferation reagent was added to the cells (culture medium 1000 µL/well DMEM/5%FCS) under sterile conditions. (1:10 final dilution). After this incubation period, the formazan dye formed is measured and quantified using a spectrophotometer (Uvikon Kontron, Neufahrn, Germany). The measured absorbance at 450 nm (reference wavelength 630 nm) correlates directly with the number of viable cells.

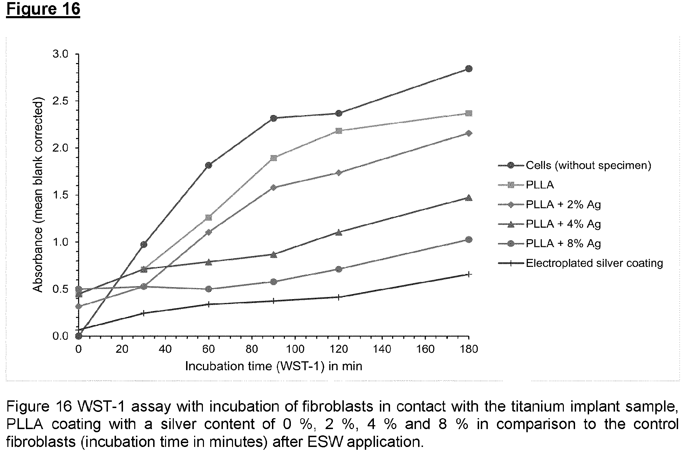

Cell proliferation of fibroblasts under the influence of different Ag concentrations was determined by WST-1 assay. The results are shown in figure 16.

### 9 Topography, Morphology and Thickness

### 9.1 Method

The topography of the coating(s) according to 1.2.2 and 1.2.3 was studied qualitatively and quantitatively. For a qualitative analysis regarding surface texture and coating coverage integrity, a scanning electron microscope (SEM) (Zeiss EVO MA10, Carl Zeiss Microscopy GmbH, Jena, Germany) in combination with the energy dispersive X-ray spectroscopy (EDS) (XFLASH^{®}6|10 Detector, Bruker Co., Billerica, Massachusetts, USA) with an accelerating voltage of *EV* = 20 kV, working distance *WD =* 11 mm and 500x, 1000x, 2000x and 5000x magnifications was used. To reduce charging of the non-conductive coatings, a thin layer of gold was previously applied on the sample surfaces using DC-sputtering (Polaron E5000, Polaron Equipment Ltd, Watford, UK) for *t =* 40 s with a current of *I* = 10 mA and a voltage of *U* = 1.17 kV. In an SEM environment, the specimens must be stable under vacuum. For coating materials that do not fulfill this requirement, a light microscope (LM) (Axio Imager, Carl Zeiss Microscopy GmbH, Jena) was used to qualitatively analyze the topography in bright field mode with 100x, 200x and 500x magnification, respectively. For a quantitative analysis, contactless surface roughness measurements were conducted using a confocal microscope (CM) (MarSurf CM mobile, Mahr GmbH, Göttingen, Germany) with a 20x lens, an evaluation length of 4 mm and a cutoff wavelength of λ_{c} = 0.8 mm in accordance with the ASTM F2791-15 standard (n = 5). The post processing was done using the MountainsMap^{®} software (Digital Surf^{®}, Besançon, France) and the widely used mean roughness indexes *Rₐ* (2D) (ISO 21920) and *Sₐ* (3D) (EN ISO 25178) was calculated. To improve the quality when testing the transparent PLLA coating, a DC-sputtering was conducted additionally for *t = 120 s.*

Both the analysis of the morphology and the layer thickness were performed microscopically using a cross-section polish with an SEM and LM according to the ISO 2808 standard. The required metallographic preparation was conducted according to the method listed in Table 4.

**Table 4: Metallographic preparation method.**

| | Grinding 1 | Grinding 2 | Polishing 1 | Polishing 2 |
|---|---|---|---|---|
| Discs / Cloths | SIPLAS Re - K400 | DIPLAS Re - K600 | PT Super Plan | PT Chem |
| Abrasive | Diamond | Diamond | Diamond-Susp. P | Silica Susp.* |
| Grain size | D40 / P400 | D60 / P600 | 9 µm | 20 nm |
| Lubricant | Water | Water | Lubricant Green | - |
| Rpm | 300 min⁻¹ | 300 min⁻¹ | 150 min⁻¹ | 150 min⁻¹ |
| Force/specimen | 30 N | 30 N | 25 N | 15 N |
| Time | Until plane | 3 min | 5 min | 8 min |

| | | | | |
|---|---|---|---|---|
| *Oxide polishing with fumed silica suspension mixed with 20% hydrogen peroxide (H₂O₂) | | | | |

To detect changes in the layer thickness over the cross-section, measurements were performed 18 times along each cross-section polish. Additionally, the thickness was determined by an eddy-current gauge (Fischerscope MMS PC2 ETA3.3, Helmut Fischer GmbH, Sindelfingen, Germany) with 18 measurements randomly distributed along the coating surface.

### 9.2 Topography, Morphology and Thickness

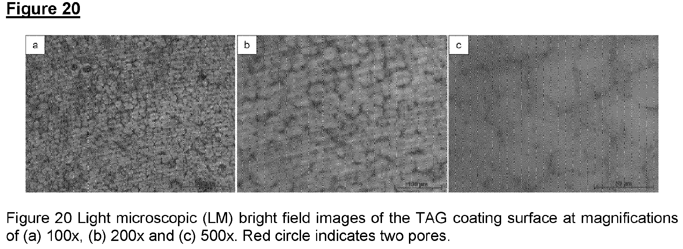

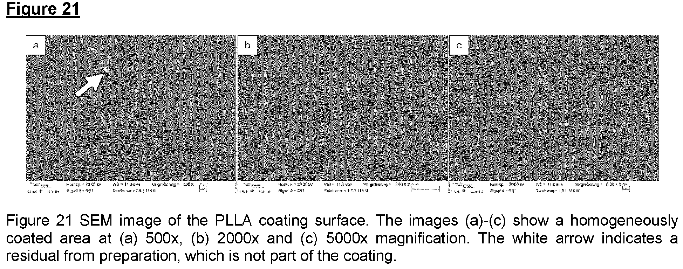

The qualitative analysis regarding surface texture and coating coverage integrity for both coatings is shown in figure 20 and figure 21, respectively. Despite two pores which are caused by the manual dip coating process, the lipid coating and the PLLA coating show a smooth surface without any defects and a homogeneous coverage of the surface, resulting in a complete protection of the titanium surface and the ability of a homogeneous elution of the incorporated active ingredients along the coated implant surface. The lipid coating displays the typical multilocular droplet structure. The particle indicated by the white arrow (figure 20) is not part of the coating but a residual from preparation.

Regarding the effect of topography on bacterial adhesion, defined surface features (e.g. patterned or line-like grooves) decrease the bacterial adhesion if their dimension is smaller than a single bacterium and vice versa (Vasudevan et al. 2014; Helbig et al. 2016). Therefore, this coating shows a favorable topography in order to prevent bacterial adhesion.

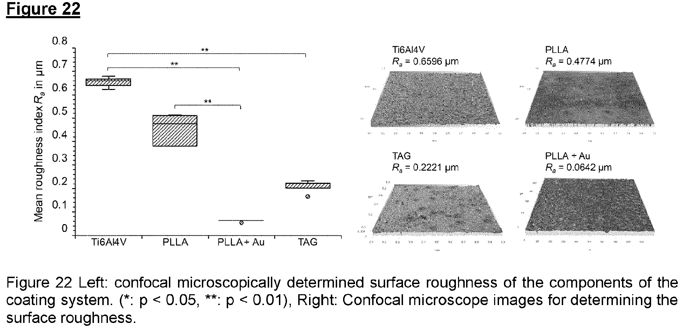

The component surface roughness of the coating system determined by confocal microscope is shown in figure 22. The lipid coating shows a highly significant lower surface roughness with a median of *Rₐ* = 0.2221 (0.0440) µm than the Ti₆Al₄V sample with *Rₐ* = 0.6596 (0.0429) µm. The previous DC-sputtering decreased the surface roughness of PLLA highly significant since the coating becomes non-transparent. The resulting surface roughness of the Au-sputtered PLLA with *Rₐ* = 0.0642 (0.0069) µm is reduced highly significant compared to the Ti₆Al₄V surface. Furthermore, the roughness of the PLLA coating is comparable to values in literature of *Rₐ* = 25 ± 3 nm (Szustakiewicz et al. 2021). A higher roughness value is associated with an enlargement of the implant surface and thus a larger surface area for potential bacterial colonization and subsequent biofilm formation (Yao et al. 2020; Yoda et al. 2014). Therefore, the coatings show preferable properties regarding the surface roughness. Furthermore, the roughness of the titanium substrate is in good accordance with the roughness reported by Guo, Matinlinna et al. *(Rₐ* = 0.448 µm) and the proximal femoral replacement prosthesis as reference part (*Rₐ* = 0.888 µm).

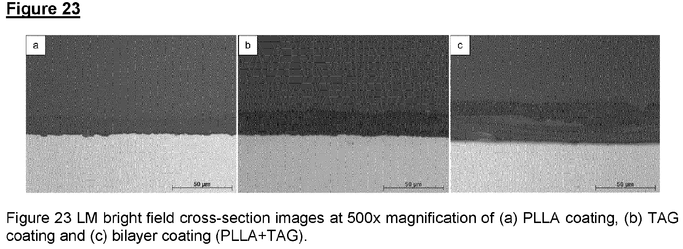

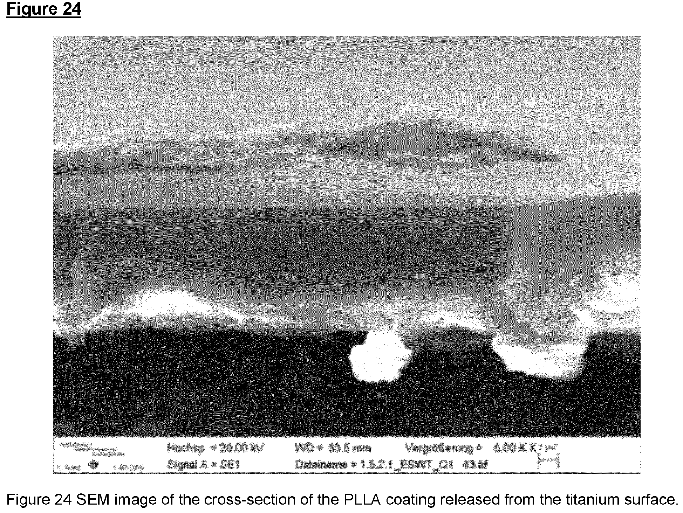

To investigate the coating's morphology, cross-section polishes were analyzed via LM and SEM (figure 23 and 24). Both coatings and the bilayer coating are fully connected to the titanium surface and the PLLA coating, respectively (figure 23). Furthermore, no pores but a homogeneously dense coating is detectable (figure 24). Therefore, this provides the requirement for the function of the coatings *in vivo*, i.e. the homogeneous distribution of active ingredients, the constancy of the antibacterial effect and the consistent ability to activate the PLLA coating.

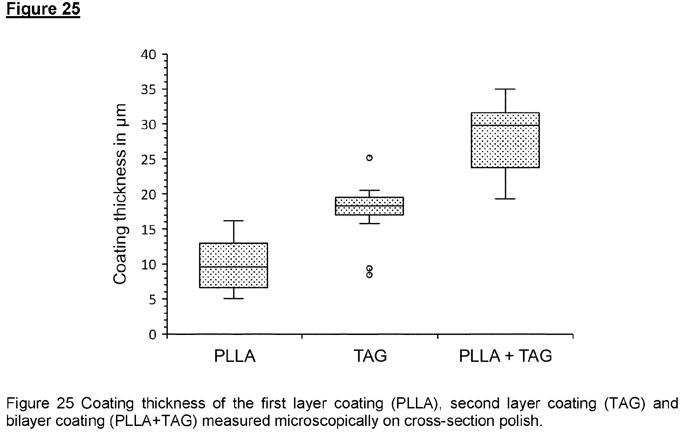

The cross-section polishes (figure 23) were also used to measure the coating thickness. These results shown in figure 25. The comparison of the two measurement principles (left) shows a highly significant deviation, which already indicates a non-homogeneous thickness distribution along the surface. Furthermore, the thickness distribution along the cross-section polish (right) confirms this observation by measuring in one plane.

### 10 Cytotoxicity

The cytotoxicity has been investigated by determining the cell count analysis for the lipid coating (figure 11). By comparing the cell proliferation of the lipid coating with the control group, a positive effect of the coating is recognized by promoting the growth of osteoblasts. Therefore, the coating could meet two essential requirements: enhanced cell proliferation and promotion of the implant integration with a simultaneous release of anti-infectives.

Cell viability was additionally investigated from a WST-1 assay with fibroblasts and a silver concentration series in PLLA. The results are shown in figure 16. All silver concentrations show a higher cell viability compared to a silver coating produced by electroplating, which is already in clinical use as approved medical device.

### 11 Antibacterial effect

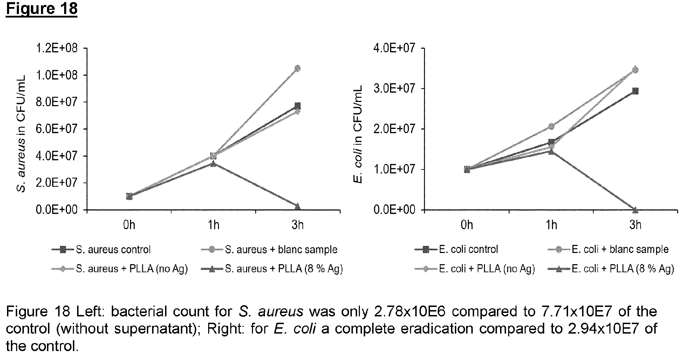

The antibacterial effect of the silver released by ESW was first tested for its biological activity in a suspension culture and inhibitory test (figure 17). To determine the antibacterial effect of the activatable PLLA coating loaded with 8% silver, suspension cultures (OD₅₇₈ 0.1) of *S*. *aureus* and *E. coli* were mixed with the supernatant after application of ESW. The effect on bacterial growth is shown in figure 18. Three hours after incubation at 37°C, the bacterial count for *S. aureus* was strongly reduced and for *Escherichia coli* a complete eradication resulted.

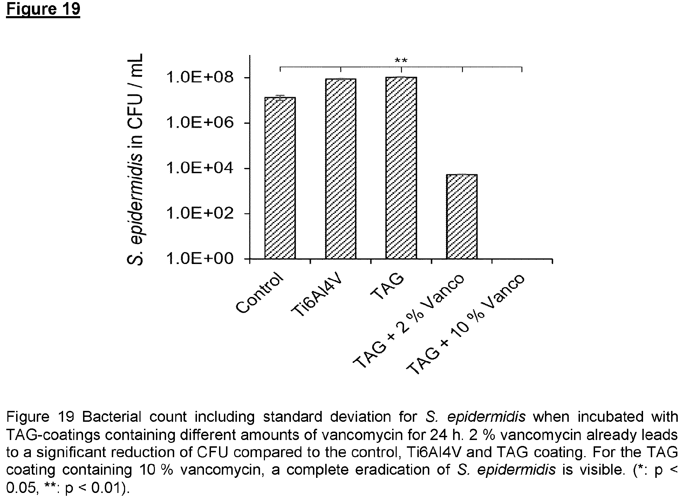

The antibacterial effect of the vancomycin loaded TAG coating on *S. epidermidis* after incubation for 24 h is shown in figure 19. While 2 % vancomycin in the TAG coating already leads to a highly significant reduction of *S. epidermidis,* for 10 % vancomycin a complete eradication is visible. The TAG coating containing vancomycin has proven the *in vitro* capability of eradicating bacteria and enhancing osteoblast growth (figure 11).

### REFERENCES

1. Gbejuade, Herbert O.; Lovering, Andrew M.; Webb, Jason C. (2015): The role of microbial biofilms in prosthetic joint infections. In: Acta orthopaedica 86 (2), S. 147-158. DOI: 10.3109/17453674.2014.966290.
2. Ong, Kevin L.; Kurtz, Steven M.; Lau, Edmund; Bozic, Kevin J.; Berry, Daniel J.; Parvizi, Javad (2009): Prosthetic joint infection risk after total hip arthroplasty in the Medicare population. In: The Journal of arthroplasty 24 (6 Suppl), S. 105-109. DOI: 10.1016/j.arth.2009.04.027
3. Gosheger, Georg; Gebert, Carsten; Ahrens, Helmut; Streitbuerger, Arne; Winkelmann, Winfried; Hardes, Jendrik (2006): Endoprosthetic reconstruction in 250 patients with sarcoma. In: Clinical orthopaedics and related research 450, S. 164-171. DOI: 10.1097/01.blo.0000223978.36831.39.
4. Funovics, Philipp T.; Hipfl, Christian; Hofstaetter, Jochen G.; Puchner, Stephan; Kotz, Rainer I.; Dominkus, Martin (2011): Management of septic complications following modular endoprosthetic reconstruction of the proximal femur. In: International Orthopaedics (SICOT) 35 (10), S. 1437-1444. DOI: 10.1007/s00264-010-1054-0.
5. Theil, C.; Röder, J.; Gosheger, G.; Deventer, N.; Dieckmann, R.; Schorn, D. et al. (2019): What is the Likelihood That Tumor Endoprostheses Will Experience a Second Complication After First Revision in Patients With Primary Malignant Bone Tumors And What Are Potential Risk Factors? In: Clinical orthopaedics and related research 477 (12), S. 2705-2714. DOI: 10.1097/CORR.0000000000000955.
6. Gosheger, Georg; Sass, Jens (2002): ENDOPROTHESE MIT GALVANISCHER SILBERSCHICHT. Angemeldet durch Implantcast GmbH am 18.02.2002. Anmeldenr: 02719863. Veröffentlichungsnr: EP000001361837B1.
7. Romanò, Carlo Luca; Scarponi, Sara; Gallazzi, Enrico; Romanò, Delia; Drago, Lorenzo (2015): Antibacterial coating of implants in orthopaedics and trauma: a classification proposal in an evolving panorama. In: Journal of orthopaedic surgery and research 10, S. 157. DOI: 10.1186/s13018-015-0294-5.
8. Hardes, Jendrik; Eiff, Christof von; Streitbuerger, Arne; Balke, Maurice; Budny, Tymoteus; Henrichs, Marcel P. et al. (2010): Reduction of periprosthetic infection with silver-coated megaprostheses in patients with bone sarcoma. In: Journal of surgical oncology 101 (5), S. 389-395. DOI: 10.1002/jso.21498.
9. Schmidt-Braekling, Tom; Streitbuerger, Arne; Gosheger, Georg; Boettner, Friedrich; Nottrott, Markus; Ahrens, Helmut et al. (2017): Silver-coated megaprostheses: review of the literature. In: European journal of orthopaedic surgery & traumatology : orthopedie traumatologie 27 (4), S. 483-489. DOI: 10.1007/s00590-017-1933-9.
10. Scoccianti, Guido; Frenos, Filippo; Beltrami, Giovanni; Campanacci, Domenico Andrea; Capanna, Rodolfo (2016): Levels of silver ions in body fluids and clinical results in silver-coated megaprostheses after tumour, trauma or failed arthroplasty. In: Injury 47 Suppl 4, S11-S16. DOI: 10.1016/j.injury.2016.07.042.
11. Karacan I, Chou J, Ben-Nissan B et al. (2018) Adhesion and Scratch Testing of Antibiotic Loaded Poly-Lactic Acid Biocomposite Thin Films on Metallic Implants. KEM 782:195-200. https://doi.org/10.4028/www.scientific.net/KEM.782.19
12. Peters M, Leyens C (eds) (2002) Titan und Titanlegierungen, [3., völlig neu bearb. Aufl.]. Wiley-VCH, Weinheim.
13. Guo CY, Matinlinna JP, Tsoi JK-H et al. (2019) Residual Contaminations of Silicon-Based Glass, Alumina and Aluminum Grits on a Titanium Surface After Sandblasting. Silicon 11:2313-2320. https://doi.org/10.1007/s12633-015-9287-6.
14. Hamed Haddad Kashani, Mathias Schmelcher, et al. (20217) - Recombinant Endolysins as Potential Therapeutics against Antibiotic-Resistant Staphylococcus aureus: Current Status of Research and Novel Delivery Strategies Clin Microbiol Rev. 2017 Nov 29;31(1):e00071-17. doi: 10.1128/CMR.00071-17
15. Ayesha Lone, Hany Anany, et al. (2016) Development of prototypes of bioactive packaging materials based on immobilized bacteriophages for control of growth of bacterial pathogens in foods; Int J Food Microbiol 2016 Jan 18;217:49-58, doi: 10.1016/j.ijfoodmicro.2015.10.011.Epub 2015 Oct 22.
16. Brennan SA, Ni Fhoghlú C, Devitt BM et al. (2015) Silver nanoparticles and their orthopaedic applications. Bone Joint J 97-B:582-589. https://doi.org/10.1302/0301-620X.97B5.33336
17. Chaloupka K, Malam Y, Seifalian AM (2010) Nanosilver as a new generation of nanoproduct in biomedical applications. Trends Biotechnol 28:580-588. https://doi.org/10.1016/j.tibtech.2010.07.006.
18. F04 Committee Guide for Assessment of Surface Texture of Non-Porous Biomaterials in Two Dimensions(ASTM F2791 - 15).
19. DIN EN ISO 21920-2:2020-04, Geometrische Produktspezifikation_(GPS)_-Oberflächenbeschaffenheit: Profile_- Teil_2: Begriffe und Parameter für die Oberflächenbeschaffenheit (ISO/DIS_21920-2:2020); Deutsche und Englische Fassung prEN_ISO_21920-2:2020
20. Deutsches Institut für Normierung (2016) Geometrische Produktspezifikation (GPS) - Oberflächenbeschaffenheit: Flächenhaft - Teil 1: Angabe von Oberflächenbeschaffenheit(DIN EN ISO 25178-1:2016-12)
21. DIN EN ISO 2808:2019-12, Beschichtungsstoffe_- Bestimmung der Schichtdicke (ISO_2808:2019); Deutsche Fassung EN_ISO_2808:2019
22. Szustakiewicz K, Kryszak B, Dzienny P et al. (2021) Cytotoxicity Study of UV-Laser-Irradiated PLLA Surfaces Subjected to Bio-Ceramisation: A New Way towards Implant Surface Modification. IJMS 22:8436. https://doi.org/10.3390/ijms22168436
23. Yao W-L, Lin JCY, Salamanca E et al. (2020) Er,Cr:YSGG Laser Performance Improves Biological Response on Titanium Surfaces. Materials (Basel) 13. https://doi.org/10.3390/ma13030756
24. Ständert V, Borcherding K, Bormann N et al. (2021) Antibiotic-loaded amphora-shaped pores on a titanium implant surface enhance osteointegration and prevent infections. Bioactive Materials 6:2331-2345. https://doi.org/10.1016/j.bioactmat.2021.01.012
25. Ren X, van der Mei HC, Ren Y et al. (2021) Antimicrobial loading of nanotubular titanium surfaces favoring surface coverage by mammalian cells over bacterial colonization. Mater Sci Eng C Mater Biol Appl 123:112021. https://doi.org/10.1016/j.msec.2021.112021
26. Rahnamaee SY, Bagheri R, Heidarpour H et al. (2021) Nanofibrillated chitosan coated highly ordered titania nanotubes array/graphene nanocomposite with improved biological characters. Carbohydrate Polymers 254:117465. https://doi.org/10.1016/j.carbpol.2020.117465
27. Kuehn K-D, Ege W, Gopp U (2005) Acrylic bone cements: composition and properties. 993 Orthop Clin North Am 36:17-28, v. https://doi.org/10.1016/j.oc1.2004.06.010
28. Vasudevan R, Kennedy AJ, Merritt M et al. (2014) Microscale patterned surfaces reduce bacterial fouling-microscopic and theoretical analysis. Colloids Surf B Biointerfaces 117:225-232. https://doi.org/10.1016/i.colsurfb.2014.02.037.
29. Helbig R, Günther D, Friedrichs J et al. (2016) The impact of structure dimensions on initial bacterial adhesion. Biomater Sci 4:1074-1078. https://doi.org/10.1039/c6bm00078a.
30. Hoffman LR, D'Argenio DA, MacCoss MJ et al. (2005) Aminoglycoside antibiotics induce bacterial biofilm formation. Nature 436:1171-1175. https://doi.org/10.1038/nature03912
31. Wang Q, Sun F-J, Liu Y et al. (2010) Enhancement of biofilm formation by subinhibitory concentrations of macrolides in icaADBC-positive and -negative clinical isolates of Staphylococcus epidermidis. Antimicrob Agents Chemother 54:2707-2711. https://doi.org/10.1128/AAC.01565-09.
32. Dunne WM (1990) Effects of subinhibitory concentrations of vancomycin or cefamandole on biofilm production by coagulase-negative staphylococci. Antimicrob Agents Chemother 34:390-393. https://doi.org/10.1128/AAC.34.3.390.
33. Schmidt-Braekling T, Streitbuerger A, Gosheger G et al. (2017) Silver-coated megaprostheses: review of the literature. Eur J Orthop Surg Traumatol 27:483-489. https://doi.org/10.1007/s00590-017-1933-9.
34. Wan AT, Conyers RA, Coombs CJ et al. (1991) Determination of silver in blood, urine, and tissues of volunteers and burn patients. Clin Chem 37:1683-1687.
35. Tweden KS, Cameron JD, Razzouk AJ et al. (1997) Biocompatibility of silver-modified polyester for antimicrobial protection of prosthetic valves. J Heart Valve Dis 6:553-561.
36. La Brutel de Riviere A, Dossche KM, Birnbaum DE et al. (2000) First clinical experience with a mechanical valve with silver coating. J Heart Valve Dis 9:123-9; discussion 129-30

## Claims

1. An implant having a surface comprising a coating on at least a portion of the surface of the implant, wherein the coating comprises
a) a first layer, the first layer comprising at least one polylactide and silver ions and
wherein
i) the silver ions are homogenously dissolved in the first layer;and
ii) optionally a second layer is present arranged on the first layer, wherein the second layer comprises at least one lipid and at least one antibiotic/antiinfective,
wherein the at least one lipid is a monoacylglycerol, a diacyglycerol, a triacylglycerol, sterol, saturated and/or unsaturated fatty acid, saturated and/or unsaturated fatty acid-salts, and/or saturated fatty alcohol, and/or saturated fatty acid ester and/or a combination thereof;
the monoacylglycerol comprises glycerol and wherein one of the three available hydroxy groups are esterified with a saturated fatty acid;
the diacyglycerol comprises glycerol wherein two of the three available hydroxy groups are esterified with a saturated fatty acid;
the triacylglycerol comprises glycerol wherein three of the three available hydroxy groups are esterified with a saturated fatty acid;
the fatty acid is R-COOH or the corresponding fatty acid salt thereof;
the fatty alcohol R-OH;
the fatty acid ester R-OR;
wherein R is (C₆-₂₃)alkyl or (C₆-₂₃)alkenyl.

2. The implant according to claim 1, wherein
i) the polylactide is a poly(L-lactide) and/or
ii) the first layer comprises of from 0.01 to 20%, preferably 0.1 to 15%, more preferably 0.5 to 10%, most preferably 1.5 to 10% by weight silver ions, particularly preferred 5 to 10 %, based on 100% by weight of the first layer; and/or
iii) the counterion is selected from a group of silver compounds that are soluble and nontoxic in non-polar solvents; and/or
iv) wherein the silver ions are provided in form of a salt or a silver complex compound; and/or
v) wherein the silver ions are provided in form of a salt and the silver salt is selected from a group consisting of silver sulfadiazine, silver diethyldithiocarbamate, silver oxide, silver carbonate and silver nitrate, silver acetate, silver benzoate, silver iodate, silver laurate, silver protein, silver chloride, and silver palmitate and any combination thereof, preferably silver diethyldithiocarbamate, more preferably silver nitrate; and/or
vi) the first layer comprises at least 70%, more preferably at least 80%, most preferably at least 90%, particularly preferred at least 95%, more particularly preferred at least 99% of the at least one polylactide;
vii) the first layer further comprises hydroxyapatite or another poorly soluble calcium compound e.g. calcium carbonate.; wherein optionally the first layer comprises 0.1 to 10% by weight hydroxyapatite, based on 100% by weight of the first layer; and/or
viii) the first layer further comprises phage-enzymes; wherein optionally the first layer comprises 0.1 to 10% by weight phage-enzymes, based on 100% by weight of the first layer; and/or
ix) the first layer has a thickness of from 1 to 50 µm.

3. The implant according to claim 1, wherein in the second layer,
a)a poly(lactide-co-glycolide) is present selected from a group of polylactides with the following properties: defined degradation rate, neutrality of influence on pH value, e.g. a poly(D,L-lactide-co-glycolide) (CAS number 1354955-03-5); and/or
b) the second layer comprises of from 0.1 to 60%, preferably 1 to 50%, more preferably 10 to 25%, by weight of the antibiotic, based on 100% by weight of the second layer; and/or
c) the antibiotic is selected from beta-lactam antibiotics (β-lactams), penicillins, cephalosporins, monobactams, carbapenems, aminoglycoside antibiotics (aminoglycosides), quinolone antibiotics (quinolones), Glycopeptide antibiotics (glycopeptides), Lincosamide antibiotics (lincosamides), Macrolide antibiotics (macrolides), Ketolide antibiotics (ketolides), Nitroimidazole derivatives (nitroimidazoles), Polypeptide antibiotics (polypeptides), Sulfonamide antibiotics (sulfonamides), Diaminopyrimidines (trimethoprim), Tetracycline antibiotics (tetracyclines), Oxazolidinone antibiotics (oxazolidinones), lipopeptide antibiotics (lipopeptides), others such as rifampicin, chloramphenicol, tigecycline, mupirocin, fosfomycin and other anti-infective, antibiotic bactericidal, bacteriostatic substances, a group consisting of vancomycin, daptomycin, rifampicin, fosfomycin, gentamycin, and any combination thereof; and/or
d) the second layer has a thickness of from 1 to 50 µm; and/or
e) the second layer further comprises a saccharide, preferably the saccharide is at least one of maltotetraose, maltotriose, maltodextrine or combinations thereof and/or the second layer comprises of from 0.1 to 10%, more preferably 0.1 to 5%, most preferably 0.1 to 1,5%, particularly preferred 0.1 to 1 %, by weight of the saccharide, based on 100% by weight of the second layer; and/or
f) the second layer further comprises phage-enzymes; wherein optionally the second layer comprises 0.1 to 10% by weight phage-enzymes, based on 100% by weight of the second layer; and/or
g) the second layer has a thickness of from 1 to 50 µm.

4. The implant according to any one of claims 1 to 3, wherein the coating further comprises a third layer arranged on the second layer wherein the third layer comprises at least one lipid and at least one antibiotic/antiinfective and/or silver ions; wherein optionally
a) the lipid is a monoacylglycerol, diacyglycerol, triacylglycerol, sterol, saturated and/or unsaturated fatty acid, saturated and/or unsaturated fatty acid-salts, and/or saturated fatty alcohol, and/or saturated fatty acid ester and/or a combination thereof; and/or
b) the third layer comprises of from 0.1 to 60%, preferably 1 to 50%, more preferably 10 to 25% by weight of the antibiotic, based on 100% by weight of the third layer; and/or
c) the antibiotic is selected from beta-lactam antibiotics (β-lactams), penicillins, cephalosporins, monobactams, carbapenems, aminoglycoside antibiotics (aminoglycosides), quinolone antibiotics (quinolones), Glycopeptide antibiotics (glycopeptides), Lincosamide antibiotics (lincosamides), Macrolide antibiotics (macrolides), Ketolide antibiotics (ketolides), Nitroimidazole derivatives (nitroimidazoles), Polypeptide antibiotics (polypeptides), Sulfonamide antibiotics (sulfonamides), Diaminopyrimidines (trimethoprim), Tetracycline antibiotics (tetracyclines), Oxazolidinone antibiotics (oxazolidinones), lipopeptide antibiotics (lipopeptides), others such as rifampicin, chloramphenicol, tigecycline, mupirocin, fosfomycin and other anti-infective, antibiotic bactericidal, bacteriostatic substances, a group consisting of vancomycin, daptomycin, rifampicin, fosfomycin, gentamycin, and any combination thereof and/or
d) the third layer further comprises a saccharide; preferably the saccharide is at least one of maltotetraose, maltotriose, maltodextrine or combinations thereof; and/or
e) the third layer comprises of from 0.1 to 10% by weight of the saccharide, more preferably 0.1 to 5%, most preferably 0.1 to 1,5%, particularly preferred 0.1 to 1 %, based on 100% by weight of the third layer and/or
f) the first layer further comprises phage-enzymes; wherein optionally the third layer comprises 0.1 to 10% by weight phage-enzymes, based on 100% by weight of the third layer; and/or
g) the third layer has a thickness of from 1 to 50 µm.

5. The implant according to any one of the preceding claims, wherein the surface of the implant comprises
i) a metal and/or, preferably a metal; more preferably the surface of the implant comprises 316LVM, CoCrMo, titanium or a titanium alloy; most preferably the titanium alloy is Ti₆Al₄V and/or
ii) a non-metal, preferably a polymer, more preferably a polymer consisting of the group polyether ether ketone (PEEK), ultra-High molecular weight polyethylene (UHMWPE), low density polyethylene (LDPE), polymethylmethacrylate (PMMA), or polyphenylsulfone (PPSU). More preferably, the polymer is PEEK.

6. The implant according to any one of the preceding claims, wherein
i) a silver metal layer is arranged between the surface of the implant and the first layer; and/or
ii) a calcium oxalate layer is arranged between the surface of the implant and the first layer; and/or
iii) the surface of the implant to be coated is treated with glass bead blasting; and/or
iv) a TiN layer is arranged between the surface; and/or
v) an intermediate layer is selected from the group consisting of PVD-layers (physical vapour deposition), TPS-layers (titanium plasma spray), calcium phosphate layers, layers caused by etching (oxidation, passivation, roughening), anodization, and a silver metal layer or any combination thereof is arranged between the surface of the implant and the first layer; and/or
vi) the implant is selected from a group comprising an implantable prosthesis, in particular a hip prosthesis, a shoulder prosthesis, an elbow prosthesis, a knee prosthesis, or an implant for trauma, maxillo-facial, or spinal surgery such as e.g. a screw or a plate, a nail or a rod, intervertebral spacers, as well as others including but not limited to cardiological and neurosurgical implants such as shunts, electrodes, valves, stents, and catheters.

7. A method of manufacturing an implant, preferably according to any one of the preceding claims, wherein the method comprises forming
a first layer arranged on at least a portion of a surface of the implant, said forming of the first layer comprising contacting at a least a portion of the surface of the implant with a first coating composition comprising a polylactide and silver ions and
wherein the coating composition comprises pyridine.

8. The method according to claim 7, wherein the method comprises forming a second layer arranged on the first layer, said forming of the second layer comprising contacting the first layer with a second coating composition comprising at least one lipid and at least one antiinfective/antibiotic,
wherein the at least one lipid is a monoacylglycerol, a diacyglycerol, a triacylglycerol, sterol, saturated and/or unsaturated fatty acid, saturated and/or unsaturated fatty acid-salts, and/or saturated fatty alcohol, and/or saturated fatty acid ester and/or a combination thereof;
the monoacylglycerol comprises glycerol and wherein one of the three available hydroxy groups are esterified with a saturated fatty acid;
the diacyglycerol comprises glycerol wherein two of the three available hydroxy groups are esterified with a saturated fatty acid;
the triacylglycerol comprises glycerol wherein three of the three available hydroxy groups are esterified with a saturated fatty acid;
the fatty acid is R-COOH or the corresponding fatty acid salt thereof;
the fatty alcohol R-OH;
the fatty acid ester R-OR;
wherein R is (C₆-₂₃)alkyl or (C₆-₂₃)alkenyl.

9. The method according to claim 7, wherein
i) the polylactide is a poly(L-lactide); and/or
ii) the silver ions are provided in form of a silver salt or silver complex; preferably the silver ions are provided in form of a salt and the silver salt is selected from a group consisting of silver sulfadiazine, silver diethyldithiocarbamate, silver oxide, silver carbonate and silver nitrate, silver acetate, silver benzoate, silver iodate, silver laurate, silver protein, silver chloride, and silver palmitate and any combination thereof; and/or
iii) the first coating composition further comprises hydroxyapatite or another poorly soluble calcium compound e.g. calcium carbonate.; and/or
iv) the first coating composition comprises a solvent; preferably the solvent is chloroform or pyridine; preferably chloroform and/or
v) at least a portion of the surface of the implant is contacted with the first coating composition by dip coating or spray coating and/or
vi) the forming of the first layer further comprises drying and/or
vii) the method further comprises forming a second layer arranged on the first layer, said forming of the second layer comprising contacting the first layer with a second coating composition comprising at least one poly(lactide-co-glycolide) and at least one antiinfective/antibiotic and/or silver ions optionally
a) the poly(lactide-co-glycolide) is a poly(D,L-lactide-co-glycolide); and/or
b) the antibiotic is selected from beta-lactam antibiotics (β-lactams), penicillins, cephalosporins, monobactams, carbapenems, aminoglycoside antibiotics (aminoglycosides), quinolone antibiotics (quinolones), Glycopeptide antibiotics (glycopeptides), Lincosamide antibiotics (lincosamides), Macrolide antibiotics (macrolides), Ketolide antibiotics (ketolides), Nitroimidazole derivatives (nitroimidazoles), Polypeptide antibiotics (polypeptides), Sulfonamide antibiotics (sulfonamides), Diaminopyrimidines (trimethoprim), Tetracycline antibiotics (tetracyclines), Oxazolidinone antibiotics (oxazolidinones), lipopeptide antibiotics (lipopeptides), others such as rifampicin, chloramphenicol, tigecycline, mupirocin, fosfomycin and other anti-infective, antibiotic bactericidal, bacteriostatic substances, a group consisting of vancomycin, daptomycin, rifampicin, fosfomycin, and any combination thereof; and/or
c) the second coating composition comprises a solvent, preferably the solvent is dimethyl sulfoxide; and/or
d) the first layer is contacted with the second coating composition by dip coating or spray coating; and/or
e) the forming of the second layer further comprises drying.

10. The method according to any one of claims 7 or 9, wherein the method further comprises forming a second layer arranged on the first layer, said forming of the second layer comprising contacting the first layer with a second coating composition comprising at least one lipid and at least one antiinfective/antibiotic and/or silver ions; wherein optionally
a) the lipid is a monoacylglycerol, diacyglycerol, triacylglycerol, sterol, saturated and/or unsaturated fatty acid, saturated and/or unsaturated fatty acid-salts, and/or saturated fatty alcohol, and/or saturated fatty acid ester and/or a combination thereof and/or
b) the antibiotic is selected from beta-lactam antibiotics (β-lactams), penicillins, cephalosporins, monobactams, carbapenems, aminoglycoside antibiotics (aminoglycosides), quinolone antibiotics (quinolones), Glycopeptide antibiotics (glycopeptides), Lincosamide antibiotics (lincosamides), Macrolide antibiotics (macrolides), Ketolide antibiotics (ketolides), Nitroimidazole derivatives (nitroimidazoles), Polypeptide antibiotics (polypeptides), Sulfonamide antibiotics (sulfonamides), Diaminopyrimidines (trimethoprim), Tetracycline antibiotics (tetracyclines), Oxazolidinone antibiotics (oxazolidinones), lipopeptide antibiotics (lipopeptides), others such as rifampicin, chloramphenicol, tigecycline, mupirocin, fosfomycin and other anti-infective, antibiotic bactericidal, bacteriostatic substances, a group consisting of vancomycin, daptomycin, rifampicin, fosfomycin, gentamycin, and any combination thereof and/or
c) the second coating composition further comprises a saccharide, preferably the saccharide is, is at least one of maltotetraose, maltotriose, maltodextrine or combinations thereof; and/or
d) the second coating composition comprises a solvent; preferably the solvent is pentane; and/or
e) the first layer is contacted with the second coating composition by dip coating or spray coating; and/or
f) the forming of the second layer further comprises drying.

11. The method according to any one of claims 8 to 11, wherein the method further comprises forming a third layer arranged on the second layer, said forming of the third layer comprising contacting the second layer with a third coating composition comprising at least one lipid and at least one antiinfective/antibiotic and/or silver ions wherein optionally
a) the at least one lipid is a monoacylglycerol, diacyglycerol, triacylglycerol, sterol, saturated and/or unsaturated fatty acid, saturated and/or unsaturated fatty acid-salts, and/or saturated fatty alcohol, and/or saturated fatty acid ester and/or a combination thereof; and/or
b) the antibiotic is selected from beta-lactam antibiotics (β-lactams), penicillins, cephalosporins, monobactams, carbapenems, aminoglycoside antibiotics (aminoglycosides), quinolone antibiotics (quinolones), Glycopeptide antibiotics (glycopeptides), Lincosamide antibiotics (lincosamides), Macrolide antibiotics (macrolides), Ketolide antibiotics (ketolides), Nitroimidazole derivatives (nitroimidazoles), Polypeptide antibiotics (polypeptides), Sulfonamide antibiotics (sulfonamides), Diaminopyrimidines (trimethoprim), Tetracycline antibiotics (tetracyclines), Oxazolidinone antibiotics (oxazolidinones), lipopeptide antibiotics (lipopeptides), others such as rifampicin, chloramphenicol, tigecycline, mupirocin, fosfomycin and other anti-infective, antibiotic bactericidal, bacteriostatic substances, a group consisting of vancomycin, daptomycin, rifampicin, fosfomycin, gentamycin and any combination thereof; and/or
c) the third coating composition further comprises a saccharide; preferably the saccharide is at least one of maltotetraose, maltotriose, maltodextrine or combinations thereof; and/or
d) the third coating composition comprises a solvent; preferably the solvent is pentane; and/or
e) the second layer is contacted with the third coating composition by dip coating or spray coating; and/or.
f) the forming of the third layer further comprises drying.

12. The method according to any one of claims 7 to 11, wherein
A)
a) the surface of the implant comprises a metal and/or a non-metal preferably the surface of the implant comprises a metal such as, CoCrMo, 316 LVM implant steel, TiN, coated Cr-Co-Mo titanium or a titanium alloy, more preferably Ti₆Al₄V or
b) the surface of the implant comprises a polymer, preferably, the surface of the implant comprises a polymer consisting of the group polyether ether ketone (PEEK), ultra-High molecular weight polyethylene (UHMWPE), low density polyethylene (LDPE), polymethylmethacrylate (PMMA), or polyphenylsulfone (PPSU) and/or
B)
i) a silver metal layer is arranged between the surface of the implant and the first layer or
ii) a calcium oxalate layer is applied on the surface of the implant before forming the first layer or
iii) the surface of the implant to be coated is treated with glass bead blasting; and/or
C) the implant is selected from a group comprising an implantable prosthesis, in particular a hip prosthesis, a shoulder prosthesis, an elbow prosthesis, a knee prosthesis, or an implant for trauma surgery such as e.g., a screw or a plate, a nail or a rod, intervertebral spacers, as well as others including but not limited to cardiological and neurosurgical implants (shunts, electrodes, valves, stents, catheters, etc.).

13. An implant, obtainable by a method according to any one of claims 7 to 12.

14. A coating of an implant as defined in claims 1 to 6 for use in a method for preventing or treating a local infection with a pathogen near the implanted implant exhibiting said coating as defined in claims 1 to 6, wherein the method comprises exposure of the first layer of the coating, comprising the first coating composition, of the implanted implant to acoustic shock waves, wherein the pressure gradient of one shock wave is 1 to 1500 MPa/mm resulting in an increased release of silver ions from the first layer of the coating,
wherein optionally
ii) the peak pressure is 10 to 150 MPa:
iii) the pressure gradient is 10 to 150 MPa/mm; and/or
iv) the impulse duration is 10 to 10000 ns, more preferably 100 to 1000 ns, more preferably 150 to 500 ns; and/or
v) the shock wave transmission into the tissue is 0.1 mm to 1 m, more preferably 1 mm to 500 mm, most preferably 10 to 200 mm; and/or
vi) the energy of one shock wave is 0.1 to 100 mJ, more preferably 1 to 50 mJ, most preferably 10 to 40 mJ; and/or
vii) the positive pressure is major to the negative pressure component of one shock wave;
viii) the shock wave transfers an energy which is sufficient to overcome the tensile strength of the coating material of the first layer to the surface of the implant, resulting in removal of at least a part of the first layer from the implant, wherein the tensile strength is 5 to 70 MPa, preferably 10-60 MPa;
ix) the energy flux density of the shock waves is 0.1 to 20 mJ/mm², preferably 0.5 to 10 mJ/mm², more preferably 1 to 5 mJ/mm², most preferably 1.1 to 3 mJ/mm².

## Patentansprüche

1. Implantat mit einer Oberfläche, die auf mindestens einem Teil der Oberfläche des Implantats eine Beschichtung aufweist, wobei die Beschichtung Folgendes umfasst a) eine erste Schicht, wobei die erste Schicht mindestens ein Polylactid und Silberionen umfasst, und
wobei
i) die Silberionen homogen in der ersten Schicht gelöst sind; und
ii) gegebenenfalls eine zweite Schicht vorhanden ist, die auf der ersten Schicht angeordnet ist, wobei die zweite Schicht mindestens ein Lipid und mindestens ein Antibiotikum/Antiinfektivum umfasst,
wobei das mindestens eine Lipid ein Monoacylglycerin, ein Diacylglycerin, ein Triacylglycerin, ein Sterol, eine gesättigte und/oder ungesättigte Fettsäure, Salze gesättigter und/oder ungesättigter Fettsäuren und/oder einen gesättigten Fettalkohol und/oder einen gesättigten Fettsäureester und/oder eine Kombination davon ist;
das Monoacylglycerin Glycerin umfasst und wobei eine der drei verfügbaren Hydroxylgruppen mit einer gesättigten Fettsäure verestert ist;
das Diacylglycerin Glycerin umfasst, wobei zwei der drei verfügbaren Hydroxylgruppen mit einer gesättigten Fettsäure verestert sind;
das Triacylglycerin umfasst Glycerin, wobei drei der drei verfügbaren Hydroxylgruppen mit einer gesättigten Fettsäure verestert sind;
die Fettsäure ist R-COOH oder das entsprechende Fettsäuresalz davon;
der Fettalkohol R-OH;
der Fettsäureester R-OR;
wobei R (C₆ -₂₃ )Alkyl oder (C₆ -₂₃ )Alkenyl ist.

2. Das Implantat nach Anspruch 1, wobei
i) das Polylactid ein Poly(L-Lactid) ist und/oder
ii) die erste Schicht 0,01 bis 20 Gew.-%, vorzugsweise 0,1 bis 15 Gew.-%, noch bevorzugter 0,5 bis 10 Gew.-%, am meisten bevorzugt 1,5 bis 10 Gew.-% Silberionen, besonders bevorzugt 5 bis 10 Gew.-%, bezogen auf 100 Gew.-% der ersten Schicht, enthält; und/oder
iii) das Gegenion aus einer Gruppe von Silberverbindungen ausgewählt ist, die in unpolaren Lösungsmitteln löslich und ungiftig sind; und/oder
iv) wobei die Silberionen in Form eines Salzes oder einer Silberkomplexverbindung vorliegen; und/oder
v) wobei die Silberionen in Form eines Salzes vorliegen und das Silbersalz aus einer Gruppe ausgewählt ist, die aus Silbersulfadiazin, Silberdiethyldithiocarbamat, Silberoxid, Silbercarbonat und Silbernitrat, Silberacetat, Silberbenzoat, Silberjodat, Silberlaurat, Silberprotein, Silberchlorid und Silberpalmitat sowie beliebigen Kombinationen davon, vorzugsweise Silberdiethyldithiocarbamat, noch bevorzugter Silbernitrat; und/oder
vi) die erste Schicht mindestens 70 %, vorzugsweise mindestens 80 %, am meisten bevorzugt mindestens 90 %, besonders bevorzugt mindestens 95 %, am meisten besonders bevorzugt mindestens 99 % des mindestens einen Polylactids umfasst;
vii) die erste Schicht umfasst ferner Hydroxylapatit oder eine andere schwer lösliche Calciumverbindung, z. B. Calciumcarbonat; wobei die erste Schicht optional 0,1 bis 10 Gew.-% Hydroxylapatit, bezogen auf 100 Gew.-% der ersten Schicht, umfasst; und/oder
viii) die erste Schicht umfasst ferner Phagenenzyme; wobei die erste Schicht optional 0,1 bis 10 Gew.-% Phagenenzyme, bezogen auf 100 Gew.-% der ersten Schicht, umfasst; und/oder
ix) die erste Schicht eine Dicke von 1 bis 50 µm aufweist.

3. Implantat nach Anspruch 1, wobei in der zweiten Schicht
a) ein Poly(lactid-co-glycolid) vorhanden ist, das aus einer Gruppe von Polylactiden mit den folgenden Eigenschaften ausgewählt ist: definierte Abbaurate, neutraler Einfluss auf den pH-Wert, z. B. ein Poly(D,L-lactid-co-glycolid) (CAS-Nummer 1354955-03-5); und/oder
b) die zweite Schicht 0,1 bis 60 Gew.-%, vorzugsweise 1 bis 50 Gew.-%, noch bevorzugter 10 bis 25 Gew.-% des Antibiotikums, bezogen auf 100 Gew.-% der zweiten Schicht, umfasst; und/oder
c) das Antibiotikum wird ausgewählt aus Beta-Lactam-Antibiotika (β-Lactame), Penicillinen, Cephalosporinen, Monobactamen, Carbapenemen, Aminoglykosid-Antibiotika (Aminoglykoside), Chinolon-Antibiotika (Chinolone), Glykopeptid-Antibiotika (Glykopeptide), Lincosamid-Antibiotika (Lincosamide), Makrolid-Antibiotika (Makrolide), Ketolid-Antibiotika (Ketolide), Nitroimidazol-Derivate (Nitroimidazole), Polypeptid-Antibiotika (Polypeptide), Sulfonamid-Antibiotika (Sulfonamide), Diaminopyrimidine (Trimethoprim), Tetracyclin-Antibiotika (Tetracycline), Oxazolidinon-Antibiotika (Oxazolidinone), Lipopeptid-Antibiotika (Lipopeptide), weitere wie Rifampicin, Chloramphenicol, Tigecyclin, Mupirocin, Fosfomycin und andere antiinfektiöse, antibiotische, bakterizide und bakteriostatische Substanzen, eine Gruppe bestehend aus Vancomycin, Daptomycin, Rifampicin, Fosfomycin, Gentamycin und beliebigen Kombinationen davon; und/oder
d) die zweite Schicht eine Dicke von 1 bis 50 µm aufweist; und/oder
e) die zweite Schicht umfasst ferner ein Saccharid, wobei das Saccharid vorzugsweise mindestens eines der folgenden ist: Maltotetraose, Maltotriose, Maltodextrin oder Kombinationen davon ist und/oder die zweite Schicht 0,1 bis 10 Gew.-%, vorzugsweise 0,1 bis 5 Gew.-%, am bevorzugtesten 0,1 bis 1,5 Gew.-%, besonders bevorzugt 0,1 bis 1 Gew.-% des Saccharids, bezogen auf 100 Gew.-% der zweiten Schicht, enthält; und/oder
f) die zweite Schicht ferner Phagenenzyme umfasst; wobei die zweite Schicht gegebenenfalls 0,1 bis 10 Gew.-% Phagenenzyme, bezogen auf 100 Gew.-% der zweiten Schicht, umfasst; und/oder
g) die zweite Schicht eine Dicke von 1 bis 50 µm aufweist.

4. Das Implantat nach einem der Ansprüche 1 bis 3, wobei die Beschichtung ferner eine auf der zweiten Schicht angeordnete dritte Schicht umfasst, wobei die dritte Schicht mindestens ein Lipid und mindestens ein Antibiotikum/Antiinfektivum und/oder Silberionen umfasst; wobei optional
a) das Lipid ein Monoacylglycerin, Diacylglycerin, Triacylglycerin, Sterin, eine gesättigte und/oder ungesättigte Fettsäure, Salze gesättigter und/oder ungesättigter Fettsäuren und/oder einen gesättigten Fettalkohol und/oder einen gesättigten Fettsäureester und/oder eine Kombination davon ist; und/oder
b) die dritte Schicht 0,1 bis 60 Gew.-%, vorzugsweise 1 bis 50 Gew.-%, noch bevorzugter 10 bis 25 Gew.-% des Antibiotikums, bezogen auf 100 Gew.-% der dritten Schicht, umfasst; und/oder
c) das Antibiotikum ausgewählt ist aus Beta-Lactam-Antibiotika (β-Lactame), Penicillinen, Cephalosporinen, Monobactamen, Carbapenemen, Aminoglykosid-Antibiotika (Aminoglykoside), Chinolon-Antibiotika (Chinolone), Glykopeptid-Antibiotika (Glykopeptide), Lincosamid-Antibiotika (Lincosamide), Makrolid-Antibiotika (Makrolide), Ketolid-Antibiotika (Ketolide), Nitroimidazol-Derivate (Nitroimidazole), Polypeptid-Antibiotika (Polypeptide), Sulfonamid-Antibiotika (Sulfonamide), Diaminopyrimidine (Trimethoprim), Tetracyclin-Antibiotika (Tetracycline), Oxazolidinon-Antibiotika (Oxazolidinone), Lipopeptid-Antibiotika (Lipopeptide), weitere wie Rifampicin, Chloramphenicol, Tigecyclin, Mupirocin, Fosfomycin und andere antiinfektiöse, antibiotische, bakterizide und bakteriostatische Substanzen, eine Gruppe bestehend aus Vancomycin, Daptomycin, Rifampicin, Fosfomycin, Gentamycin und beliebigen Kombinationen davon und/oder
d) die dritte Schicht ferner ein Saccharid umfasst; vorzugsweise ist das Saccharid mindestens eines von Maltotetraose, Maltotriose, Maltodextrin oder Kombinationen davon; und/oder
e) die dritte Schicht 0,1 bis 10 Gew.-% des Saccharids, vorzugsweise 0,1 bis 5 %, am meisten bevorzugt 0,1 bis 1,5 %, besonders bevorzugt 0,1 bis 1 %, bezogen auf 100 Gew.-% der dritten Schicht, umfasst und/oder
f) die erste Schicht ferner Phagenenzyme umfasst; wobei die dritte Schicht gegebenenfalls 0,1 bis 10 Gew.-% Phagenenzyme, bezogen auf 100 Gew.-% der dritten Schicht, umfasst; und/oder
g) die dritte Schicht eine Dicke von 1 bis 50 µm aufweist.

5. Das Implantat nach einem der vorstehenden Ansprüche, wobei die Oberfläche des Implantats
i) ein Metall und/oder, vorzugsweise, ein Metall; noch bevorzugter umfasst die Oberfläche des Implantats 316LVM, CoCrMo, Titan oder eine Titanlegierung; am meisten bevorzugt ist die Titanlegierung Ti₆Al₄V und/oder
ii) ein Nichtmetall, vorzugsweise ein Polymer, noch bevorzugter ein Polymer aus der Gruppe Polyetheretherketon (PEEK), ultrahochmolekulares Polyethylen (UHMWPE), Polyethylen niedriger Dichte (LDPE), Polymethylmethacrylat (PMMA) oder Polyphenylsulfon (PPSU). Noch bevorzugter ist das Polymer PEEK.

6. Das Implantat nach einem der vorstehenden Ansprüche, wobei
i) zwischen der Oberfläche des Implantats und der ersten Schicht eine Silbermetallschicht angeordnet ist; und/oder
ii) zwischen der Oberfläche des Implantats und der ersten Schicht eine Calciumoxalatschicht angeordnet ist; und/oder
iii) die zu beschichtende Oberfläche des Implantats durch Glasperlstrahlen behandelt wird; und/oder
iv) eine TiN-Schicht zwischen der Oberfläche angeordnet ist; und/oder
v) eine Zwischenschicht aus der Gruppe ausgewählt ist, die aus PVD-Schichten (Physical Vapor Deposition), TPS-Schichten (Titanium Plasma Spray), Calciumphosphatschichten, durch Ätzen (Oxidation, Passivierung, Aufrauen) erzeugten Schichten, Anodisierung und einer Silbermetallschicht besteht, oder eine beliebige Kombination davon zwischen der Oberfläche des Implantats und der ersten Schicht angeordnet ist; und/oder
vi) das Implantat aus einer Gruppe ausgewählt ist, die eine implantierbare Prothese, insbesondere eine Hüftprothese, eine Schulterprothese, eine Ellenbogenprothese, eine Knieprothese oder ein Implantat für die Unfall-, Kiefer- und Gesichtschirurgie oder die Wirbelsäulenchirurgie umfasst, wie z. B. eine Schraube oder eine Platte, einen Nagel oder eine Stange, Zwischenwirbelspacer sowie weitere, einschließlich, aber nicht beschränkt auf kardiologische und neurochirurgische Implantate wie Shunts, Elektroden, Ventile, Stents und Katheter.

7. Verfahren zur Herstellung eines Implantats, vorzugsweise gemäß einem der vorstehenden Ansprüche, wobei das Verfahren das Ausbilden
einer ersten Schicht, die auf mindestens einem Teil einer Oberfläche des Implantats angeordnet ist, wobei das Ausbilden der ersten Schicht das Inkontaktbringen mindestens eines Teils der Oberfläche des Implantats mit einer ersten Beschichtungszusammensetzung umfasst, die ein Polylactid und Silberionen enthält, und
wobei die Beschichtungszusammensetzung Pyridin umfasst.

8. Das Verfahren nach Anspruch 7, wobei das Verfahren das Bilden einer zweiten Schicht umfasst, die auf der ersten Schicht angeordnet ist, wobei das Bilden der zweiten Schicht das Inkontaktbringen der ersten Schicht mit einer zweiten Beschichtungszusammensetzung umfasst, die mindestens ein Lipid und mindestens ein Antiinfektivum/Antibiotikum enthält, wobei das mindestens eine Lipid ein Monoacylglycerin, ein Diacylglycerin, ein Triacylglycerin, ein Sterin, eine gesättigte und/oder ungesättigte Fettsäure, Salze gesättigter und/oder ungesättigter Fettsäuren und/oder ein gesättigter Fettalkohol und/oder ein gesättigter Fettsäureester und/oder eine Kombination davon ist;
das Monoacylglycerin Glycerin umfasst und wobei eine der drei verfügbaren Hydroxylgruppen mit einer gesättigten Fettsäure verestert ist;
das Diacylglycerin Glycerin umfasst, wobei zwei der drei verfügbaren Hydroxylgruppen mit einer gesättigten Fettsäure verestert sind;
das Triacylglycerin umfasst Glycerin, wobei drei der drei verfügbaren Hydroxylgruppen mit einer gesättigten Fettsäure verestert sind;
die Fettsäure ist R-COOH oder das entsprechende Fettsäuresalz davon;
der Fettalkohol R-OH;
der Fettsäureester R-OR;
wobei R (C₆-₂₃ )Alkyl oder (C₆-₂₃ )Alkenyl ist.

9. Das Verfahren nach Anspruch 7, wobei
i) das Polylactid ein Poly(L-lactid) ist; und/oder
ii) die Silberionen in Form eines Silbersalzes oder eines Silberkomplexes vorliegen; vorzugsweise die Silberionen in Form eines Salzes vorliegen und das Silbersalz aus einer Gruppe ausgewählt ist, die aus Silbersulfadiazin, Silberdiethyldithiocarbamat, Silberoxid, Silbercarbonat und Silbernitrat, Silberacetat, Silberbenzoat, Silberiodat, Silberlaurat, Silberprotein, Silberchlorid und Silberpalmitat sowie beliebigen Kombinationen davon besteht; und/oder
iii) die erste Beschichtungszusammensetzung umfasst ferner Hydroxylapatit oder eine andere schwer lösliche Calciumverbindung, z. B. Calciumcarbonat; und/oder
iv) die erste Beschichtungszusammensetzung ein Lösungsmittel enthält; vorzugsweise ist das Lösungsmittel Chloroform oder Pyridin; vorzugsweise Chloroform und/oder
v) mindestens ein Teil der Oberfläche des Implantats durch Tauchbeschichtung oder Sprühbeschichtung mit der ersten Beschichtungszusammensetzung in Kontakt gebracht wird und/oder
vi) das Aufbringen der ersten Schicht umfasst ferner das Trocknen und/oder
vii) das Verfahren umfasst ferner die Bildung einer zweiten Schicht, die auf der ersten Schicht angeordnet ist, wobei die Bildung der zweiten Schicht das Inkontaktbringen der ersten Schicht mit einer zweiten Beschichtungszusammensetzung umfasst, die mindestens ein Poly(lactid-co-glycolid) und mindestens ein Antiinfektivum/Antibiotikum und/oder Silberionen enthält, gegebenenfalls
a) das Poly(lactid-co-glycolid) ein Poly(D,L-lactid-co-glycolid) ist; und/oder
b) das Antibiotikum ausgewählt ist aus Beta-Lactam-Antibiotika (β-Lactamen), Penicillinen, Cephalosporinen, Monobactamen, Carbapenemen, Aminoglykosid-Antibiotika (Aminoglykosiden), Chinolon-Antibiotika (Chinolonen), Glykopeptid-Antibiotika (Glykopeptide), Lincosamid-Antibiotika (Lincosamide), Makrolid-Antibiotika (Makrolide), Ketolid-Antibiotika (Ketolide), Nitroimidazol-Derivate (Nitroimidazole), Polypeptid-Antibiotika (Polypeptide), Sulfonamid-Antibiotika (Sulfonamide), Diaminopyrimidine (Trimethoprim), Tetracyclin-Antibiotika (Tetracycline), Oxazolidinon-Antibiotika (Oxazolidinone), Lipopeptid-Antibiotika (Lipopeptide), weitere wie Rifampicin, Chloramphenicol, Tigecyclin, Mupirocin, Fosfomycin und andere antiinfektiöse, antibiotische, bakterizide und bakteriostatische Substanzen, eine Gruppe bestehend aus Vancomycin, Daptomycin, Rifampicin, Fosfomycin und beliebigen Kombinationen davon; und/oder
c) die zweite Beschichtungszusammensetzung umfasst ein Lösungsmittel, vorzugsweise ist das Lösungsmittel Dimethylsulfoxid; und/oder
d) die erste Schicht wird durch Tauchbeschichtung oder Sprühbeschichtung mit der zweiten Beschichtungszusammensetzung in Kontakt gebracht; und/oder
e) die Bildung der zweiten Schicht umfasst ferner das Trocknen.

10. Das Verfahren nach einem der Ansprüche 7 oder 9, wobei das Verfahren ferner das Bilden einer auf der ersten Schicht angeordneten zweiten Schicht umfasst, wobei das Bilden der zweiten Schicht das Inkontaktbringen der ersten Schicht mit einer zweiten Beschichtungszusammensetzung umfasst, die mindestens ein Lipid und mindestens ein Antiinfektivum/Antibiotikum und/oder Silberionen umfasst;
wobei optional
a) das Lipid ein Monoacylglycerin, Diacylglycerin, Triacylglycerin, Sterin, eine gesättigte und/oder ungesättigte Fettsäure, Salze gesättigter und/oder ungesättigter Fettsäuren und/oder einen gesättigten Fettalkohol und/oder einen gesättigten Fettsäureester und/oder eine Kombination davon ist und/oder
b) das Antibiotikum ausgewählt ist aus Beta-Lactam-Antibiotika (β-Lactame), Penicillinen, Cephalosporinen, Monobactamen, Carbapenemen, Aminoglykosid-Antibiotika (Aminoglykoside), Chinolon-Antibiotika (Chinolone), Glykopeptid-Antibiotika (Glykopeptide), Lincosamid-Antibiotika (Lincosamide), Makrolid-Antibiotika (Makrolide), Ketolid-Antibiotika (Ketolide), Nitroimidazol-Derivate (Nitroimidazole), Polypeptid-Antibiotika (Polypeptide), Sulfonamid-Antibiotika (Sulfonamide), Diaminopyrimidine (Trimethoprim), Tetracyclin-Antibiotika (Tetracycline), Oxazolidinon-Antibiotika (Oxazolidinone), Lipopeptid-Antibiotika (Lipopeptide), weitere wie Rifampicin, Chloramphenicol, Tigecyclin, Mupirocin, Fosfomycin und andere antiinfektiöse, antibiotische, bakterizide und bakteriostatische Substanzen, eine Gruppe bestehend aus Vancomycin, Daptomycin, Rifampicin, Fosfomycin, Gentamycin und beliebigen Kombinationen davon und/oder
c) die zweite Beschichtungszusammensetzung umfasst ferner ein Saccharid, wobei das Saccharid vorzugsweise mindestens eines von Maltotetraose, Maltotriose, Maltodextrin oder Kombinationen davon ist; und/oder
d) die zweite Beschichtungszusammensetzung umfasst ein Lösungsmittel; vorzugsweise ist das Lösungsmittel Pentan; und/oder
e) die erste Schicht durch Tauchbeschichtung oder Sprühbeschichtung mit der zweiten Beschichtungszusammensetzung in Kontakt gebracht wird; und/oder
f) die Bildung der zweiten Schicht umfasst ferner das Trocknen.

11. Das Verfahren nach einem der Ansprüche 8 bis 11, wobei das Verfahren ferner das Bilden einer dritten Schicht umfasst, die auf der zweiten Schicht angeordnet ist, wobei das Bilden der dritten Schicht das Inkontaktbringen der zweiten Schicht mit einer dritten Beschichtungszusammensetzung umfasst, die mindestens ein Lipid und mindestens ein Antiinfektivum/Antibiotikum und/oder Silberionen umfasst, wobei optional
a) das mindestens eine Lipid ein Monoacylglycerin, Diacylglycerin, Triacylglycerin, Sterin, eine gesättigte und/oder ungesättigte Fettsäure, Salze gesättigter und/oder ungesättigter Fettsäuren und/oder einen gesättigten Fettalkohol und/oder einen gesättigten Fettsäureester und/oder eine Kombination davon ist; und/oder
b) das Antibiotikum ausgewählt ist aus Beta-Lactam-Antibiotika (β-Lactame), Penicillinen, Cephalosporinen, Monobactamen, Carbapenemen, Aminoglykosid-Antibiotika (Aminoglykoside), Chinolon-Antibiotika (Chinolone), Glykopeptid-Antibiotika (Glykopeptide), Lincosamid-Antibiotika (Lincosamide), Makrolid-Antibiotika (Makrolide), Ketolid-Antibiotika (Ketolide), Nitroimidazol-Derivate (Nitroimidazole), Polypeptid-Antibiotika (Polypeptide), Sulfonamid-Antibiotika (Sulfonamide), Diaminopyrimidine (Trimethoprim), Tetracyclin-Antibiotika (Tetracycline), Oxazolidinon-Antibiotika (Oxazolidinone), Lipopeptid-Antibiotika (Lipopeptide), weitere wie Rifampicin, Chloramphenicol, Tigecyclin, Mupirocin, Fosfomycin und andere antiinfektiöse, antibiotische, bakterizide und bakteriostatische Substanzen, eine Gruppe bestehend aus Vancomycin, Daptomycin, Rifampicin, Fosfomycin, Gentamycin und beliebigen Kombinationen davon; und/oder
c) die dritte Beschichtungszusammensetzung umfasst ferner ein Saccharid; vorzugsweise ist das Saccharid mindestens eines von Maltotetraose, Maltotriose, Maltodextrin oder Kombinationen davon; und/oder
d) die dritte Beschichtungszusammensetzung umfasst ein Lösungsmittel; vorzugsweise ist das Lösungsmittel Pentan; und/oder
e) die zweite Schicht wird durch Tauchbeschichtung oder Sprühbeschichtung mit der dritten Beschichtungszusammensetzung in Kontakt gebracht; und/oder
f) die Bildung der dritten Schicht umfasst ferner das Trocknen.

12. Das Verfahren nach einem der Ansprüche 7 bis 11, wobei
A)
a) die Oberfläche des Implantats ein Metall und/oder ein Nichtmetall umfasst, vorzugsweise umfasst die Oberfläche des Implantats ein Metall wie beispielsweise CoCrMo, 316 LVM-Implantatstahl, TiN, beschichtetes Cr-Co-Mo-Titan oder eine Titanlegierung, noch bevorzugter Ti₆Al₄V oder
b) die Oberfläche des Implantats ein Polymer umfasst, vorzugsweise umfasst die Oberfläche des Implantats ein Polymer aus der Gruppe Polyetheretherketon (PEEK), ultrahochmolekulares Polyethylen (UHMWPE), Polyethylen niedriger Dichte (LDPE), Polymethylmethacrylat (PMMA) oder Polyphenylsulfon (PPSU) und/oder
B)
i) zwischen der Oberfläche des Implantats und der ersten Schicht eine Silbermetallschicht angeordnet ist oder
ii) vor der Bildung der ersten Schicht eine Calciumoxalatschicht auf die Oberfläche des Implantats aufgebracht wird oder
iii) die Oberfläche des zu beschichtenden Implantats durch Glasperlstrahlen behandelt wird; und/oder
C) das Implantat aus einer Gruppe ausgewählt ist, die eine implantierbare Prothese, insbesondere eine Hüftprothese, eine Schulterprothese, eine Ellenbogenprothese, eine Knieprothese oder ein Implantat für die Unfallchirurgie, wie z. B. eine Schraube oder eine Platte, einen Nagel oder eine Stange, Zwischenwirbelspacer sowie weitere, einschließlich, aber nicht beschränkt auf kardiologische und neurochirurgische Implantate (Shunts, Elektroden, Klappen, Stents, Katheter usw.).

13. Ein Implantat, das durch ein Verfahren gemäß einem der Ansprüche 7 bis 12 erhältlich ist.

14. Eine Beschichtung eines Implantats gemäß den Ansprüchen 1 bis 6 zur Verwendung in einem Verfahren zur Vorbeugung oder Behandlung einer lokalen Infektion mit einem Erreger in der Nähe des implantierten Implantats, das die in den Ansprüchen 1 bis 6 definierte Beschichtung aufweist, wobei das Verfahren das Aussetzen der ersten Schicht der Beschichtung, die die erste Beschichtungszusammensetzung enthält, des implantierten Implantats akustischen Stoßwellen auszusetzen, wobei der Druckgradient einer Stoßwelle 1 bis 1500 MPa/mm beträgt, was zu einer erhöhten Freisetzung von Silberionen aus der ersten Schicht der Beschichtung führt,
wobei optional
ii) der Spitzendruck 10 bis 150 MPa beträgt;
iii) der Druckgradient 10 bis 150 MPa/mm beträgt; und/oder
iv) die Impulsdauer 10 bis 10.000 ns, vorzugsweise 100 bis 1.000 ns, am bevorzugtesten 150 bis 500 ns beträgt; und/oder
v) die Stoßwellenausbreitung im Gewebe 0,1 mm bis 1 m, vorzugsweise 1 mm bis 500 mm, am meisten bevorzugt 10 bis 200 mm beträgt; und/oder
vi) die Energie einer Stoßwelle beträgt 0,1 bis 100 mJ, vorzugsweise 1 bis 50 mJ, am meisten bevorzugt 10 bis 40 mJ; und/oder
vii) der Überdruck gegenüber der Unterdruckkomponente einer Stoßwelle überwiegt;
viii) die Stoßwelle überträgt eine Energie auf die Oberfläche des Implantats, die ausreicht, um die Zugfestigkeit des Beschichtungsmaterials der ersten Schicht zu überwinden, was zur Entfernung zumindest eines Teils der ersten Schicht vom Implantat führt, wobei die Zugfestigkeit 5 bis 70 MPa, vorzugsweise 10 bis 60 MPa, beträgt;
ix) die Energieflussdichte der Stoßwellen beträgt 0,1 bis 20 mJ/mm², vorzugsweise 0,5 bis 10 mJ/mm², noch bevorzugter 1 bis 5 mJ/mm², am meisten bevorzugt 1,1 bis 3 mJ/mm².

## Revendications

1. Implant présentant une surface comprenant un revêtement sur au moins une partie de la surface de l'implant, dans lequel le revêtement comprend
a) une première couche, ladite première couche comprenant au moins un polylactide et des ions d'argent, et
dans lequel
i) les ions d'argent sont dissous de manière homogène dans la première couche ; et
ii) éventuellement, une deuxième couche est présente, disposée sur la première couche, ladite deuxième couche comprenant au moins un lipide et au moins un antibiotique/anti-infectieux,
dans lequel ledit au moins un lipide est un monoacylglycérol, un diacylglycérol, un triacylglycérol, un stérol, un acide gras saturé et/ou insaturé, des sels d'acides gras saturés et/ou insaturés, et/ou un alcool gras saturé, et/ou un ester d'acide gras saturé et/ou une combinaison de ceux-ci ;
le monoacylglycérol comprend du glycérol et dans lequel l'un des trois groupes hydroxy disponibles est estérifié avec un acide gras saturé ;
le diacylglycérol comprend du glycérol, dans lequel deux des trois groupes hydroxy disponibles sont estérifiés par un acide gras saturé ;
le triacylglycérol comprend du glycérol dans lequel trois des trois groupes hydroxy disponibles sont estérifiés par un acide gras saturé ;
l'acide gras est R-COOH ou le sel d'acide gras correspondant ;
l'alcool gras R-OH ;
l'ester d'acide gras R-OR ;
où R est un groupe alkyle en C₆à _{C(23)}ou alcényle en C₆à _{C(23)}.

2. L'implant selon la revendication 1, dans lequel
i) le polylactide est un poly(L-lactide) et/ou
ii) la première couche comprend de 0,01 à 20 %, de préférence de 0,1 à 15 %, plus préférablement de 0,5 à 10 %, et de manière encore plus préférable de 1,5 à 10 % en poids d'ions argent, de manière particulièrement préférée de 5 à 10 %, par rapport à 100 % en poids de la première couche ; et/ou
iii) le contre-ion est choisi parmi un groupe de composés d'argent qui sont solubles et non toxiques dans des solvants non polaires ; et/ou
iv) dans lequel les ions argent sont présents sous forme d'un sel ou d'un composé complexe d'argent ; et/ou
v) dans laquelle les ions d'argent se présentent sous la forme d'un sel et le sel d'argent est choisi parmi un groupe comprenant la sulfadiazine d'argent, le diéthyldithiocarbamate d'argent, l'oxyde d'argent, le carbonate d'argent, le nitrate d'argent, l'acétate d'argent, le benzoate d'argent, l'iodate d'argent, le laurate d'argent, la protéine d'argent, le chlorure d'argent et le palmitate d'argent, ainsi que toute combinaison de ceux-ci, de préférence le diéthyldithiocarbamate d'argent, et plus préférentiellement le nitrate d'argent ; et/ou
vi) la première couche comprend au moins 70 %, de préférence au moins 80 %, de manière encore plus préférée au moins 90 %, de manière particulièrement préférée au moins 95 %, et de manière plus particulièrement préférée au moins 99 % dudit au moins un polylactide ;
vii) la première couche comprend en outre de l'hydroxyapatite ou un autre composé de calcium peu soluble, par exemple du carbonate de calcium ; la première couche pouvant éventuellement comprendre de 0,1 à 10 % en poids d'hydroxyapatite, par rapport à 100 % en poids de la première couche ; et/ou
viii) la première couche comprend en outre des enzymes de phages ; la première couche comprenant éventuellement de 0,1 à 10 % en poids d'enzymes de phages, par rapport à 100 % en poids de la première couche ; et/ou
ix) la première couche présente une épaisseur comprise entre 1 et 50 µm.

3. Implant selon la revendication 1, dans lequel, dans la deuxième couche,
a) est présent un poly(lactide-co-glycolide) choisi parmi un groupe de polylactides présentant les propriétés suivantes : vitesse de dégradation définie, absence d'influence sur le pH, par exemple un poly(D,L-lactide-co-glycolide) (numéro CAS 1354955-03-5) ; et/ou
b) la deuxième couche comprend de 0,1 à 60 %, de préférence de 1 à 50 %, mieux encore de 10 à 25 %, en poids de l'antibiotique, par rapport à 100 % en poids de la deuxième couche ; et/ou
c) l'antibiotique est choisi parmi les antibiotiques bêta-lactamines (β-lactamines), les pénicillines, les céphalosporines, les monobactames, les carbapénèmes, les antibiotiques aminoglycosides (aminoglycosides), les antibiotiques quinolones (quinolones), antibiotiques glycopeptidiques (glycopeptides), antibiotiques lincosamides (lincosamides), antibiotiques macrolides (macrolides), antibiotiques kétolides (kétolides), dérivés du nitroimidazole (nitroimidazoles), antibiotiques polypeptidiques (polypeptides), antibiotiques sulfamides (sulfamides), diaminopyrimidines (triméthoprime), antibiotiques tétracyclines (tétracyclines), antibiotiques oxazolidinones (oxazolidinones), antibiotiques lipopeptidiques (lipopeptides), d'autres tels que la rifampicine, le chloramphénicol, la tigécycline, la mupirocine, la fosfomycine et d'autres substances anti-infectieuses, antibiotiques bactéricides ou bactériostatiques, un groupe constitué de la vancomycine, de la daptomycine, de la rifampicine, de la fosfomycine, de la gentamycine et de toute combinaison de celles-ci ; et/ou
d) la deuxième couche présente une épaisseur comprise entre 1 et 50 µm ; et/ou
e) la deuxième couche comprend en outre un saccharide, de préférence le saccharide est au moins l'un parmi le maltotétraose, le maltotriose, la maltodextrine ou des combinaisons de ceux-ci ; et/ou la deuxième couche comprend de 0,1 à 10 %, de préférence de 0,1 à 5 %, plus préférablement de 0,1 à 1,5 %, et particulièrement de préférence de 0,1 à 1 % en poids du saccharide, par rapport à 100 % en poids de la deuxième couche ; et/ou
f) la deuxième couche comprend en outre des enzymes de phages ; la deuxième couche comprenant éventuellement de 0,1 à 10 % en poids d'enzymes de phages, par rapport à 100 % en poids de la deuxième couche ; et/ou
g) la deuxième couche présente une épaisseur comprise entre 1 et 50 µm.

4. Implant selon l'une quelconque des revendications 1 à 3, dans lequel l'enrobage comprend en outre une troisième couche disposée sur la deuxième couche, la troisième couche comprenant au moins un lipide et au moins un antibiotique/anti-infectieux et/ou des ions d'argent ; dans lequel, éventuellement
a) le lipide est un monoacylglycérol, un diacylglycérol, un triacylglycérol, un stérol, un acide gras saturé et/ou insaturé, des sels d'acides gras saturés et/ou insaturés, et/ou un alcool gras saturé, et/ou un ester d'acide gras saturé et/ou une combinaison de ceux-ci ; et/ou
b) la troisième couche comprend de 0,1 à 60 %, de préférence de 1 à 50 %, et de manière plus préférentielle de 10 à 25 % en poids de l'antibiotique, par rapport à 100 % en poids de la troisième couche ; et/ou
c) l'antibiotique est choisi parmi les antibiotiques bêta-lactamines (β-lactamines), les pénicillines, les céphalosporines, les monobactames, les carbapénèmes, les antibiotiques aminoglycosides (aminoglycosides), les antibiotiques quinolones (quinolones), antibiotiques glycopeptidiques (glycopeptides), antibiotiques lincosamides (lincosamides), antibiotiques macrolides (macrolides), antibiotiques kétolides (kétolides), dérivés du nitroimidazole (nitroimidazoles), antibiotiques polypeptidiques (polypeptides), antibiotiques sulfamides (sulfamides), diaminopyrimidines (triméthoprime), antibiotiques tétracyclines (tétracyclines), antibiotiques oxazolidinones (oxazolidinones), antibiotiques lipopeptidiques (lipopeptides), d'autres tels que la rifampicine, le chloramphénicol, la tigécycline, la mupirocine, la fosfomycine et d'autres substances anti-infectieuses, antibiotiques bactéricides ou bactériostatiques, un groupe constitué de la vancomycine, de la daptomycine, de la rifampicine, de la fosfomycine, de la gentamycine et de toute combinaison de celles-ci et/ou
d) la troisième couche comprend en outre un saccharide ; de préférence, le saccharide est au moins l'un parmi le maltotétraose, le maltotriose, la maltodextrine ou des combinaisons de ceux-ci ; et/ou
e) la troisième couche comprend de 0,1 à 10 % en poids du saccharide, de préférence de 0,1 à 5 %, de manière encore plus préférée de 0,1 à 1,5 %, et de manière particulièrement préférée de 0,1 à 1 %, par rapport à 100 % en poids de la troisième couche ; et/ou
f) la première couche comprend en outre des enzymes de phages ; la troisième couche comprenant éventuellement de 0,1 à 10 % en poids d'enzymes de phages, par rapport à 100 % en poids de la troisième couche ; et/ou
g) la troisième couche présente une épaisseur comprise entre 1 et 50 µm.

5. Implant selon l'une quelconque des revendications précédentes, dans lequel la surface de l'implant comprend
i) un métal et/ou, de préférence, un métal ; plus préférablement, la surface de l'implant comprend du 316LVM, du CoCrMo, du titane ou un alliage de titane ; de manière encore plus préférable, l'alliage de titane est du Ti₆Al₄V et/ou
ii) un non-métal, de préférence un polymère, plus préférablement un polymère choisi parmi le groupe comprenant le polyétheréthercétone (PEEK), le polyéthylène à ultra-haut poids moléculaire (UHMWPE), le polyéthylène basse densité (LDPE), le polyméthacrylate de méthyle (PMMA) ou le polyphénylsulfone (PPSU). Plus préférablement, le polymère est du PEEK.

6. Implant selon l'une quelconque des revendications précédentes, dans lequel
i) une couche de métal d'argent est disposée entre la surface de l'implant et la première couche ; et/ou
ii) une couche d'oxalate de calcium est disposée entre la surface de l'implant et la première couche ; et/ou
iii) la surface de l'implant à revêtir est traitée par sablage aux billes de verre ; et/ou
iv) une couche de TiN est disposée entre la surface ; et/ou
v) une couche intermédiaire est choisie parmi le groupe constitué des couches PVD (dépôt physique en phase vapeur), des couches TPS (projection de plasma de titane), des couches de phosphate de calcium, des couches obtenues par gravure (oxydation, passivation, rugosification), de l'anodisation, et une couche de métal d'argent ou toute combinaison de celles-ci est disposée entre la surface de l'implant et la première couche ; et/ou
vi) l'implant est choisi parmi un groupe comprenant une prothèse implantable, en particulier une prothèse de hanche, une prothèse d'épaule, une prothèse de coude, une prothèse de genou, ou un implant destiné à la chirurgie traumatologique, maxillo-faciale ou rachidienne, tel que, par exemple, une vis ou une plaque, un clou ou une tige, des espaceurs intervertébraux, ainsi que d'autres, y compris, sans s'y limiter, des implants cardiologiques et neurochirurgicaux tels que des shunts, des électrodes, des valves, des stents et des cathéters.

7. Procédé de fabrication d'un implant, de préférence selon l'une quelconque des revendications précédentes, dans lequel le procédé comprend la formation
une première couche disposée sur au moins une partie d'une surface de l'implant, ladite formation de la première couche comprenant la mise en contact d'au moins une partie de la surface de l'implant avec une première composition de revêtement comprenant un polylactide et des ions d'argent, et
dans lequel la composition de revêtement comprend de la pyridine.

8. Procédé selon la revendication 7, dans lequel le procédé comprend la formation d'une deuxième couche disposée sur la première couche, ladite formation de la deuxième couche comprenant la mise en contact de la première couche avec une deuxième composition de revêtement comprenant au moins un lipide et au moins un agent anti-infectieux/antibiotique, dans lequel ledit au moins un lipide est un monoacylglycérol, un diacylglycérol, un triacylglycérol, un stérol, un acide gras saturé et/ou insaturé, des sels d'acides gras saturés et/ou insaturés, et/ou un alcool gras saturé, et/ou un ester d'acide gras saturé et/ou une combinaison de ceux-ci ;
le monoacylglycérol comprend du glycérol et dans lequel l'un des trois groupes hydroxy disponibles est estérifié avec un acide gras saturé ;
le diacylglycérol comprend du glycérol, dans lequel deux des trois groupes hydroxy disponibles sont estérifiés par un acide gras saturé ;
le triacylglycérol comprend du glycérol dans lequel trois des trois groupes hydroxy disponibles sont estérifiés par un acide gras saturé ;
l'acide gras est R-COOH ou le sel d'acide gras correspondant ;
l'alcool gras R-OH ;
l'ester d'acide gras R-OR ;
où R représente un groupe alkyle (C₆₋₂₃) ou alcényle (C₆-₂₃).

9. Procédé selon la revendication 7, dans lequel
i) le polylactide est un poly(L-lactide); et/ou
ii) les ions argent sont apportés sous forme d'un sel d'argent ou d'un complexe d'argent ; de préférence, les ions argent sont fournis sous forme d'un sel et le sel d'argent est choisi parmi un groupe comprenant la sulfadiazine d'argent, le diéthyldithiocarbamate d'argent, l'oxyde d'argent, le carbonate d'argent, le nitrate d'argent, l'acétate d'argent, le benzoate d'argent, l'iodate d'argent, le laurate d'argent, la protéine d'argent, le chlorure d'argent et le palmitate d'argent, ainsi que toute combinaison de ceux-ci ; et/ou
iii) la première composition de revêtement comprend en outre de l'hydroxyapatite ou un autre composé de calcium peu soluble, par exemple du carbonate de calcium ; et/ou
iv) la première composition de revêtement comprend un solvant ; de préférence, le solvant est le chloroforme ou la pyridine ; de préférence le chloroforme et/ou
v) au moins une partie de la surface de l'implant est mise en contact avec la première composition de revêtement par enduction par immersion ou par pulvérisation ; et/ou
vi) la formation de la première couche comprend en outre un séchage et/ou
vii) le procédé comprend en outre la formation d'une deuxième couche disposée sur la première couche, ladite formation de la deuxième couche comprenant la mise en contact de la première couche avec une deuxième composition de revêtement comprenant au moins un poly(lactide-co-glycolide) et au moins un agent anti-infectieux/antibiotique et/ou des ions d'argent, éventuellement
a) le poly(lactide-co-glycolide) est un poly(D,L-lactide-co-glycolide) ; et/ou
b) l'antibiotique est choisi parmi les antibiotiques bêta-lactamines (β-lactamines), les pénicillines, les céphalosporines, les monobactames, les carbapénèmes, les antibiotiques aminoglycosides (aminoglycosides), les antibiotiques quinolones (quinolones), antibiotiques glycopeptidiques (glycopeptides), antibiotiques lincosamides (lincosamides), antibiotiques macrolides (macrolides), antibiotiques cétolides (cétolides), dérivés du nitroimidazole (nitroimidazoles), antibiotiques polypeptidiques (polypeptides), antibiotiques sulfamides (sulfamides), diaminopyrimidines (triméthoprime), antibiotiques tétracyclines (tétracyclines), antibiotiques oxazolidinones (oxazolidinones), antibiotiques lipopeptidiques (lipopeptides), d'autres tels que la rifampicine, le chloramphénicol, la tigécycline, la mupirocine, la fosfomycine et d'autres substances anti-infectieuses, antibiotiques bactéricides ou bactériostatiques, un groupe constitué de la vancomycine, de la daptomycine, de la rifampicine, de la fosfomycine et de toute combinaison de celles-ci ; et/ou
c) la deuxième composition de revêtement comprend un solvant, de préférence le diméthylsulfoxyde; et/ou
d) la première couche est mise en contact avec la deuxième composition de revêtement par enduction par immersion ou par pulvérisation; et/ou
e) la formation de la deuxième couche comprend en outre un séchage.

10. Procédé selon l'une quelconque des revendications 7 ou 9, dans lequel le procédé comprend en outre la formation d'une deuxième couche disposée sur la première couche, ladite formation de la deuxième couche comprenant la mise en contact de la première couche avec une deuxième composition de revêtement comprenant au moins un lipide et au moins un agent anti-infectieux/antibiotique et/ou des ions d'argent;
dans lequel, éventuellement
a) le lipide est un monoacylglycérol, un diacylglycérol, un triacylglycérol, un stérol, un acide gras saturé et/ou insaturé, des sels d'acides gras saturés et/ou insaturés, et/ou un alcool gras saturé, et/ou un ester d'acide gras saturé et/ou une combinaison de ceux-ci et/ou
b) l'antibiotique est choisi parmi les antibiotiques bêta-lactamines (β-lactamines), les pénicillines, les céphalosporines, les monobactames, les carbapénèmes, les antibiotiques aminoglycosides (aminoglycosides), les antibiotiques quinolones (quinolones), antibiotiques glycopeptidiques (glycopeptides), antibiotiques lincosamides (lincosamides), antibiotiques macrolides (macrolides), antibiotiques kétolides (kétolides), dérivés du nitroimidazole (nitroimidazoles), antibiotiques polypeptidiques (polypeptides), antibiotiques sulfamides (sulfamides), diaminopyrimidines (triméthoprime), antibiotiques tétracyclines (tétracyclines), antibiotiques oxazolidinones (oxazolidinones), antibiotiques lipopeptidiques (lipopeptides), d'autres tels que la rifampicine, le chloramphénicol, la tigécycline, la mupirocine, la fosfomycine et d'autres substances anti-infectieuses, antibiotiques bactéricides ou bactériostatiques, un groupe constitué de la vancomycine, de la daptomycine, de la rifampicine, de la fosfomycine, de la gentamycine et de toute combinaison de celles-ci et/ou
c) la deuxième composition d'enrobage comprend en outre un saccharide, de préférence le saccharide est au moins l'un parmi le maltotétraose, le maltotriose, la maltodextrine ou des combinaisons de ceux-ci; et/ou
d) la deuxième composition d'enrobage comprend un solvant ; de préférence, le solvant est le pentane ; et/ou
e) la première couche est mise en contact avec la deuxième composition d'enrobage par enrobage par immersion ou par pulvérisation ; et/ou
f) la formation de la deuxième couche comprend en outre un séchage.

11. Procédé selon l'une quelconque des revendications 8 à 11, dans lequel le procédé comprend en outre la formation d'une troisième couche disposée sur la deuxième couche, ladite formation de la troisième couche comprenant la mise en contact de la deuxième couche avec une troisième composition de revêtement comprenant au moins un lipide et au moins un agent anti-infectieux/antibiotique et/ou des ions d'argent, dans laquelle, éventuellement,
a) ledit au moins un lipide est un monoacylglycérol, un diacylglycérol, un triacylglycérol, un stérol, un acide gras saturé et/ou insaturé, des sels d'acides gras saturés et/ou insaturés, et/ou un alcool gras saturé, et/ou un ester d'acide gras saturé et/ou une combinaison de ceux-ci ; et/ou
b) l'antibiotique est choisi parmi les antibiotiques bêta-lactamines (β-lactamines), les pénicillines, les céphalosporines, les monobactames, les carbapénèmes, les antibiotiques aminoglycosides (aminoglycosides), les antibiotiques quinolones (quinolones), antibiotiques glycopeptidiques (glycopeptides), antibiotiques lincosamides (lincosamides), antibiotiques macrolides (macrolides), antibiotiques kétolides (kétolides), dérivés du nitroimidazole (nitroimidazoles), antibiotiques polypeptidiques (polypeptides), antibiotiques sulfamides (sulfamides), diaminopyrimidines (triméthoprime), antibiotiques tétracyclines (tétracyclines), antibiotiques oxazolidinones (oxazolidinones), antibiotiques lipopeptidiques (lipopeptides), d'autres tels que la rifampicine, le chloramphénicol, la tigécycline, la mupirocine, la fosfomycine et d'autres substances anti-infectieuses, antibiotiques bactéricides ou bactériostatiques, un groupe constitué de la vancomycine, de la daptomycine, de la rifampicine, de la fosfomycine, de la gentamycine et de toute combinaison de celles-ci ; et/ou
c) la troisième composition d'enrobage comprend en outre un saccharide ; de préférence, le saccharide est au moins l'un parmi le maltotétraose, le maltotriose, la maltodextrine ou des combinaisons de ceux-ci ; et/ou
d) la troisième composition d'enrobage comprend un solvant ; de préférence, le solvant est le pentane ; et/ou
e) la deuxième couche est mise en contact avec la troisième composition d'enrobage par enrobage par immersion ou par pulvérisation ; et/ou
f) la formation de la troisième couche comprend en outre un séchage.

12. Procédé selon l'une quelconque des revendications 7 à 11, dans lequel
A)
a) la surface de l'implant comprend un métal et/ou un non-métal ; de préférence, la surface de l'implant comprend un métal tel que CoCrMo, de l'acier chirurgical 316 LVM, du TiN, du titane revêtu de Cr-Co-Mo ou un alliage de titane, de préférence encore du Ti₆Al₄V ou
b) la surface de l'implant comprend un polymère ; de préférence, la surface de l'implant comprend un polymère choisi parmi le groupe comprenant le polyéther éther cétone (PEEK), le polyéthylène à ultra-haut poids moléculaire (UHMWPE), le polyéthylène basse densité (LDPE), le polyméthacrylate de méthyle (PMMA) ou le polyphénylsulfone (PPSU) et/ou
B)
i) une couche de métal d'argent est disposée entre la surface de l'implant et la première couche, ou
ii) une couche d'oxalate de calcium est appliquée sur la surface de l'implant avant la formation de la première couche ou
iii) la surface de l'implant à revêtir est traitée par grenaillage aux billes de verre ; et/ou
C) l'implant est choisi parmi un groupe comprenant une prothèse implantable, en particulier une prothèse de hanche, une prothèse d'épaule, une prothèse de coude, une prothèse de genou, ou un implant destiné à la chirurgie traumatologique, tel que, par exemple, une vis ou une plaque, un clou ou une tige, des espaceurs intervertébraux, ainsi que d'autres, y compris, sans s'y limiter, des implants cardiologiques et neurochirurgicaux (dérivations, électrodes, valves, stents, cathéters, etc.).

13. Implant pouvant être obtenu par un procédé selon l'une quelconque des revendications 7 à 12.

14. Revêtement d'un implant tel que défini dans les revendications 1 à 6, destiné à être utilisé dans un procédé visant à prévenir ou à traiter une infection locale par un agent pathogène à proximité de l'implant implanté présentant ledit revêtement tel que défini dans les revendications 1 à 6, dans lequel le procédé comprend l'exposition de la première couche du revêtement, comprenant la première composition de revêtement, de l'implant implanté à des ondes de choc acoustiques, le gradient de pression d'une onde de choc étant compris entre 1 et 1 500 MPa/mm, ce qui entraîne une libération accrue d'ions d'argent à partir de la première couche du revêtement,
dans laquelle, éventuellement,
ii) la pression de crête est comprise entre 10 et 150 MPa ;
iii) le gradient de pression est compris entre 10 et 150 MPa/mm ; et/ou
iv) la durée de l'impulsion est comprise entre 10 et 10 000 ns, de préférence entre 100 et 1 000 ns, et plus préférablement entre 150 et 500 ns ; et/ou
v) la pénétration de l'onde de choc dans le tissu est comprise entre 0,1 mm et 1 m, de préférence entre 1 mm et 500 mm, et de manière encore plus préférable entre 10 et 200 mm ; et/ou
vi) l'énergie d'une onde de choc est comprise entre 0,1 et 100 mJ, de préférence entre 1 et 50 mJ, et de manière encore plus préférable entre 10 et 40 mJ ; et/ou
vii) la composante de pression positive est prédominante par rapport à la composante de pression négative d'une onde de choc ;
viii) l'onde de choc transfère à la surface de l'implant une énergie suffisante pour surmonter la résistance à la traction du matériau de revêtement de la première couche, ce qui entraîne l'élimination d'au moins une partie de la première couche de l'implant, la résistance à la traction étant comprise entre 5 et 70 MPa, de préférence entre 10 et 60 MPa ;
ix) la densité de flux d'énergie des ondes de choc est comprise entre 0,1 et 20 mJ/mm², de préférence entre 0,5 et 10 mJ/mm², plus préférablement entre 1 et 5 mJ/mm², et de manière encore plus préférable entre 1,1 et 3 mJ/mm².
